# EUROPEAN PATENT APPLICATION

(11) **EP 3 848 039 A1**
(43) Date of publication of application: **14.07.2021**
(21) Application number: 19869492.9
(22) Date of filing: 30.08.2019
(51) Int. Cl.: A61K 31/7088, A61K 31/4439, A61K 45/06, A61P 43/00, A23L 33/10, A23L 33/13, G01N 33/68

(54) **PHARMACEUTICAL COMPOSITION FOR PREVENTING OR TREATING FABRY DISEASE, CONTAINING TSP1 PROTEIN INHIBITOR AS ACTIVE INGREDIENT**

(30) Priority: 04.10.2018 KR 20180118430
(71) Applicant: Korea Advanced Institute of Science and Technology, Daejeon 34141 (KR)
(72) Inventor: HAN, Yong Mahn, Daejeon 34141 (KR); DO, Hyo Sang, Daejeon 34141 (KR)
(74) Representative: EP&C
(86) International application number: PCT/KR2019/011199
(87) International publication number: WO 2020/071641

(57) **Abstract**

The present invention relates to a pharmaceutical composition for preventing or treating Fabry disease, containing a TSP1 protein inhibitor as an active ingredient. Particularly, in vascular endothelial cells produced by knocking out a TSP1 gene in induced pluripotent stem cells derived from a Fabry disease patient, of the present invention, the recovery of cell morphology, a decrease in the expression of a TSP1 gene, a decrease in the expression levels of a TSP1 protein and a phosphorylated-SMAD protein, which are anti-angiogenic factors, and an increase in the expression levels of a KDR protein and an eNOS protein, which are angiogenic factors, have been confirmed, and thus a TSP1 gene expression inhibitor or a TSP1 protein activity inhibitor can be effectively used in the treatment of Fabry disease.

<110>     Korea Advanced Institute of Science and Technology

<120>     Pharmaceutical composition for preventing or treating Fabry
          disease comprising inhibitors of TSP1 protein as an active
          ingradient

<130>     2018P-05-054

<150>     KR 10-2018-0118430
<151>     2018-10-04

<160>     6

<170>     KoPatentIn 3.0

<210>     1
<211>     7775
<212>     DNA
<213>     Homo sapiens

<400>     1
agctggcctg cgagttcagg gctcctgtcg ctctccagga gcaacctcta ctccggacgc        60

acaggcattc cccgcgcccc tccagccctc gccgccctcg ccaccgctcc cggccgccgc       120

gctccggtac acacaggatc cctgctgggc accaacagct ccaccatggg gctggcctgg       180

ggactaggcg tcctgttcct gatgcatgtg tgtggcacca accgcattcc agagtctggc       240

ggagacaaca gcgtgtttga catctttgaa ctcaccgggg ccgcccgcaa ggggtctggg       300

cgccgactgg tgaagggccc cgacccttcc agcccagctt tccgcatcga ggatgccaac       360

ctgatccccc ctgtgcctga tgacaagttc caagacctgg tggatgctgt gcgggcagaa       420

aagggtttcc tccttctggc atccctgagg cagatgaaga agacccgggg cacgctgctg       480

gccctggagc ggaaagacca ctctggccag gtcttcagcg tggtgtccaa tggcaaggcg       540

ggcaccctgg acctcagcct gaccgtccaa ggaaagcagc acgtggtgtc tgtggaagaa       600

gctctcctgg caaccggcca gtggaagagc atcaccctgt ttgtgcagga agacagggcc       660

cagctgtaca tcgactgtga aaagatggag aatgctgagt tggacgtccc catccaaagc       720

gtcttcacca gagacctggc cagcatcgcc agactccgca tcgcaaaggg gggcgtcaat       780

gacaatttcc agggggtgct gcagaatgtg aggtttgtct ttggaaccac accagaagac       840

atcctcagga acaaaggctg ctccagctct accagtgtcc tcctcaccct tgacaacaac       900

gtggtgaatg gttccagccc tgccatccgc actaactaca ttggccacaa gacaaaggac       960

ttgcaagcca tctgcggcat ctcctgtgat gagctgtcca gcatggtcct ggaactcagg      1020

ggcctgcgca ccattgtgac cacgctgcag gacagcatcc gcaaagtgac tgaagagaac      1080

aaagagttgg ccaatgagct gaggcggcct cccctatgct atcacaacgg agttcagtac      1140

agaaataacg aggaatggac tgttgatagc tgcactgagt gtcactgtca gaactcagtt      1200

accatctgca aaaaggtgtc ctgccccatc atgccctgct ccaatgccac agttcctgat      1260

ggagaatgct gtcctcgctg ttggcccagc gactctgcgg acgatggctg gtctccatgg 1320

tccgagtgga cctcctgttc tacgagctgt ggcaatggaa ttcagcagcg cggccgctcc 1380

tgcgatagcc tcaacaaccg atgtgagggc tcctcggtcc agacacggac ctgccacatt 1440

caggagtgtg acaagagatt taaacaggat ggtggctgga gccactggtc cccgtggtca 1500

tcttgttctg tgacatgtgg tgatggtgtg atcacaagga tccggctctg caactctccc 1560

agcccccaga tgaacgggaa accctgtgaa ggcgaagcgc gggagaccaa agcctgcaag 1620

aaagacgcct gccccatcaa tggaggctgg ggtccttggt caccatggga catctgttct 1680

gtcacctgtg gaggaggggt acagaaacgt agtcgtctct gcaacaaccc cacaccccag 1740

tttggaggca aggactgcgt tggtgatgta acagaaaacc agatctgcaa caagcaggac 1800

tgtccaattg atggatgcct gtccaatccc tgctttgccg gcgtgaagtg tactagctac 1860

cctgatggca gctggaaatg tggtgcttgt ccccctggtt acagtggaaa tggcatccag 1920

tgcacagatg ttgatgagtg caaagaagtg cctgatgcct gcttcaacca caatggagag 1980

caccggtgtg agaacacgga ccccggctac aactgcctgc cctgcccccc acgcttcacc 2040

ggctcacagc ccttcggcca gggtgtcgaa catgccacgg ccaacaaaca ggtgtgcaag 2100

ccccgtaacc cctgcacgga tgggacccac gactgcaaca agaacgccaa gtgcaactac 2160

ctgggccact atagcgaccc catgtaccgc tgcgagtgca agcctggcta cgctggcaat 2220

ggcatcatct gcggggagga cacagacctg gatggctggc ccaatgagaa cctggtgtgc 2280

gtggccaatg cgacttacca ctgcaaaaag gataattgcc ccaaccttcc caactcaggg 2340

caggaagact atgacaagga tggaattggt gatgcctgtg atgatgacga tgacaatgat 2400

aaaattccag atgacaggga caactgtcca ttccattaca acccagctca gtatgactat 2460

gacagagatg atgtgggaga ccgctgtgac aactgtccct acaaccacaa cccagatcag 2520

gcagacacag acaacaatgg ggaaggagac gcctgtgctg cagacattga tggagacggt 2580

atcctcaatg aacgggacaa ctgccagtac gtctacaatg tggaccagag agacactgat 2640

atggatgggg ttggagatca gtgtgacaat tgccccttgg aacacaatcc ggatcagctg 2700

gactctgact cagaccgcat tggagatacc tgtgacaaca atcaggatat tgatgaagat 2760

ggccaccaga acaatctgga caactgtccc tatgtgccca atgccaacca ggctgaccat 2820

gacaaagatg gcaagggaga tgcctgtgac cacgatgatg acaacgatgg cattcctgat 2880

gacaaggaca actgcagact cgtgcccaat cccgaccaga aggactctga cggcgatggt 2940

cgaggtgatg cctgcaaaga tgattttgac catgacagtg tgccagacat cgatgacatc 3000

tgtcctgaga atgttgacat cagtgagacc gatttccgcc gattccagat gattcctctg 3060

gaccccaaag ggacatccca aaatgaccct aactgggttg tacgccatca gggtaaagaa 3120

ctcgtccaga ctgtcaactg tgatcctgga ctcgctgtag gttatgatga gtttaatgct 3180

gtggacttca gtggcacctt cttcatcaac accgaaaggg acgatgacta tgctggattt 3240

gtctttggct accagtccag cagccgcttt tatgttgtga tgtggaagca agtcacccag 3300

tcctactggg acaccaaccc cacgagggct cagggatact cgggcctttc tgtgaaagtt 3360

gtaaactcca ccacagggcc tggcgagcac ctgcggaacg ccctgtggca cacaggaaac 3420

acccctggcc aggtgcgcac cctgtggcat gaccctcgtc acataggctg gaaagatttc 3480

accgcctaca gatggcgtct cagccacagg ccaaagacgg gtttcattag agtggtgatg 3540

tatgaaggga agaaaatcat ggctgactca ggacccatct atgataaaac ctatgctggt 3600

ggtagactag ggttgtttgt cttctctcaa gaaatggtgt tcttctctga cctgaaatac 3660

gaatgtagag atccctaatc atcaaattgt tgattgaaag actgatcata aaccaatgct 3720

ggtattgcac cttctggaac tatgggcttg agaaaacccc caggatcact tctccttggc 3780

ttccttcttt tctgtgcttg catcagtgtg gactcctaga acgtgcgacc tgcctcaaga 3840

aaatgcagtt ttcaaaaaca gactcagcat tcagcctcca atgaataaga catcttccaa 3900

gcatataaac aattgctttg gtttcctttt gaaaaagcat ctacttgctt cagttgggaa 3960

ggtgcccatt ccactctgcc tttgtcacag agcagggtgc tattgtgagg ccatctctga 4020

gcagtggact caaaagcatt ttcaggcatg tcagagaagg gaggactcac tagaattagc 4080

aaacaaaacc accctgacat cctccttcag gaacacgggg agcagaggcc aaagcactaa 4140

ggggagggcg catacccgag acgattgtat gaagaaaata tggaggaact gttacatgtt 4200

cggtactaag tcattttcag gggattgaaa gactattgct ggatttcatg atgctgactg 4260

gcgttagctg attaacccat gtaaataggc acttaaatag aagcaggaaa gggagacaaa 4320

gactggcttc tggacttcct ccctgatccc cacccttact catcacctgc agtggccaga 4380

attagggaat cagaatcaaa ccagtgtaag gcagtgctgg ctgccattgc ctggtcacat 4440

tgaaattggt ggcttcattc tagatgtagc ttgtgcagat gtagcaggaa aataggaaaa 4500

cctaccatct cagtgagcac cagctgcctc ccaaaggagg ggcagccgtg cttatatttt 4560

tatggttaca atggcacaaa attattatca acctaactaa aacattcctt ttctcttttt 4620

tcctgaatta tcatggagtt ttctaattct ctcttttgga atgtagattt tttttaaatg 4680

ctttacgatg taaaatattt attttttact tattctggaa gatctggctg aaggattatt 4740

catggaacag gaagaagcgt aaagactatc catgtcatct ttgttgagag tcttcgtgac 4800

tgtaagattg taaatacaga ttatttatta actctgttct gcctggaaat ttaggcttca 4860

tacggaaagt gtttgagagc aagtagttga catttatcag caaatctctt gcaagaacag 4920

cacaaggaaa atcagtctaa taagctgctc tgccccttgt gctcagagtg gatgttatgg 4980

gattcttttt ttctctgttt tatcttttca agtggaatta gttggttatc catttgcaaa 5040

tgttttaaat tgcaaagaaa gccatgaggt cttcaatact gttttacccc atcccttgtg 5100

catatttcca gggagaagga aagcatatac actttttctt ttcattttcc caaaagagaa 5160

aaaaatgaca aaaggtgaaa cttacataca aatattacct catttgttgt gtgactgagt 5220

aaagaatttt tggatcaagc ggaaagagtt taagtgtcta acaaacttaa agctactgta 5280

gtacctaaaa agtcagtgtt gtacatagca taaaaactct gcagagaagt attcccaata 5340

aggaaatagc attgaaatgt taaatacaat ttctgaaagt tatgtttttt ttctatcatc 5400

tggtatacca ttgctttatt tttataaatt attttctcat tgccattgga atagatatct 5460

cagattgtgt agatatgcta tttaaataat ttatcaggaa atactgcctg tagagttagt 5520

atttctattt ttatataatg tttgcacact gaattgaaga attgttggtt ttttcttttt 5580

tttgttttgt tttttttttt tttttttttt gcttttgacc tcccattttt actatttgcc 5640

aatacctttt tctaggaatg tgcttttttt tgtacacatt tttatccatt ttacattcta 5700

aagcagtgta agttgtatat tactgtttct tatgtacaag gaacaacaat aaatcatatg 5760

gaaatttata tttatactta ctgtatccat gcttatttgt tctctactgg ctttatgtca 5820

tgaagtatat gcgtaaatac cattcataaa tcaatatagc atatacaaaa ataaattaca 5880

gtaagtcata gcaacattca cagtttgtat gtgattgaga aagactgagt tgctcaggcc 5940

taggcttaga atttgctgcg tttgtggaat aaaagaacaa aatgatacat tagcctgcca 6000

tatcaaaaac atataaaaga gaaattatcc ctaagtcaag ggcccccata agaataaaat 6060

ttcttattaa ggtcattaga tgtcattgaa tccttttcaa agtgcagtat gaaaacaaag 6120

ggaaaaacac tgaagcacac gcaactctca cagcgacatt ttctgaccca cgaatgatgc 6180

cttgggtggg caacacgatt gcatgttgtg gagacacttc ggaagtaaat gtggatgagg 6240

gaggagctgt ccttgcaatg ttgagccaag cattacagat acctcctctt gaagaaggaa 6300

taataagttt aatcaaaaaa gaagactaaa aaatgtaaaa tttggaagga atccataaat 6360

gcgtgtgtgt ctaaatacaa attatcatgt gaagaaaagg cccaagtgta ccaataagca 6420

gaccttgatt tttggatggg ctaattatga atgtggaata ctgaccagtt aatttccagt 6480

tttaatgaaa acagatcaaa gaagaaattt tatgagtagg ttaaaggtct ggctttgagg 6540

tctattaaac actagaaagg actggctggg tgagataaaa tcttccttgt tgattttcac 6600

tctcattcta taaatactca tctttctgag tagccatgat cacatacaaa tgtaaattgc 6660

caaatcattt tatagtacca aggtgaagaa gcaggaacta gaaagtgttg ataatagctg 6720

tggagttagg aaaactgatg tgaaggaaat aattctttga aatggcaaag aattaaatac 6780

catcattcat tatcagaaga gttcaacgtt tgaagtgctg ggagataatt ctaattcatt 6840

cttggatagt gaagcaaaac tgattgaaaa taccaagata agacagaaaa agtgactgga 6900

aagaggagct tttcttccag gcatgttcca gtttcaccct aagactgacc ttcaaataat 6960

caggttgtac tgaaataaag gacttgttaa aaattaaaat tatgtcatcg agatgatagc 7020

ttttttcctc ctccaacagt ttattgtcat gtgttgtggg agagctcgag tgaagagcaa 7080

taaactccag gtcttataag aatgtacata caataaaggt ggtgccagca gttttttttt 7140

ttctaaagag tcacatgtag aaaagcctcc agtattaagc tcctgaattc attccttaaa 7200

taaattggct ctctctctct tctataattt ctttttcttt ttatttttga gatgaagtct 7260

tgctctgtcg cccaggctgg agtgcagtga cacaatctcg gctcactgca acctctgcct 7320

ccccggttca agcaattctc cctcctgcct cagcctccca agtagctggg actacaagcg 7380

cccgccacca agcctggcta attctgtatt tttagtaaag acggggtttc accttgttcc 7440

ggacaaacac taagccctaa agggaaatcc aaaataaaaa catctatttt taataacact 7500

ttctatctaa atcagggtga ctttttaaaa aaaatccgga agctttttgt tgaattacgt 7560

tacagactta gttaccagtc cttgttagag ttaccttcag ttgacatgct gtgaatggtc 7620

ccacctcttt tatggcagaa ttcattactt aaaataactc tattttcttc ccccttacct 7680

aaataacaga aaggctcact atgtcccaaa tatcattggc agaagcaaac tataaagtca 7740

taagcccttt gcagtgcaag tctagaaata atttt 7775

<210> 2
<211> 1170
<212> PRT
<213> Homo sapiens

<400> 2

<210>    3
<211>    27
<212>    DNA
<213>    Artificial Sequence

<220>
<223>    CRISPR/CAS9 target_GLA_1

<400>    3
tcattctttt tctcagtnnn ngattgg                                          27

<210>    4
<211>    27
<212>    DNA
<213>    Artificial Sequence

<220>
<223>    CRISPR/CAS9 target_GLA_2

<400>    4
ttctcagtnn nngattggca actttgg                                          27

<210>    5
<211>    23
<212>    DNA
<213>    Artificial Sequence

<220>
<223>    CRISPR/CAS9 target_TSP1_1

<400>    5
gtctgtggaa gaagctctcc tgg                                              23

<210>    6
<211>    23
<212>    DNA
<213>    Artificial Sequence

<220>
<223>    CRISPR/CAS9 target_TSP1_2

<400>    6
ctggagcgga aagaccactc tgg                                              23

## Description

### BACKGROUND OF THE INVENTION

### 1. Field of the Invention

The present invention relates to a pharmaceutical composition for preventing or treating Fabry disease, containing a TSP1 protein inhibitor as an active ingredient.

### 2. Description of the Related Art

Fabry disease is an X-gene-related recessive genetic disease caused by mutations in GLA, a gene encoding α-galactosidase. The GLA gene is a gene present at the xq22.1 position of the 7^{th} exon in humans, and encodes a glycoprotein composed of 370 amino acids processed from a precursor protein composed of a total of 429 amino acids (Schiffmann, R. Pharmacology & therapeutics 122, 65-77 (2009)).

Up to now, more than 400 mutation sites known to appear in the GLA gene have been reported (http://www.hgmd.cf.ac.uk), and the severity of Fabry disease symptoms varies depending on the mutation location in the GLA gene. The activity of alpha-galactosidase is not exhibited at all by most of the mutations, but residual enzyme activity of 5-10% is exhibited by some missense mutations. Patients with these mutations do not display clinically important pathophysiology (Clarke, J. T. Annals of internal medicine 146, 425-433 (2007)).

Fabry disease also shows the deficiency of a lysosomal enzyme. This enzyme deficiency is known to be due to excessive accumulation of globotriaosylceramide (Gb3, CD77), a neutral glycosphingolipid that acts as a Shiga toxin receptor in Burkitt lymphoma cells (Nudelman, E. et al. Science New York, N.Y 220, 509-511 (1983)). Accumulation of Gb3 appears in a variety of cells, such as vascular cells, cardiac cells, kidney epithelial cells, or neuronal cells in patients with Fabry disease. In particular, the accumulation of Gb3 in vascular cells causes systemic cardiovascular dysfunction such as stroke or myocardial infarction.

In the past, Fabry disease has been reported to occur 1 in 117,000 men (Meikle, P. J. et al. Jama 281, 249-254 (1999)). However, in recent years, the incidence frequency has risen rapidly, and it has been reported to occur in 1 out of 3,100 to 3,700 male children (Spada, M. et al. American journal of human genetics 79, 31-40 (2006)).

As the only way to treat Fabry disease, repetitive enzyme replacement therapy is used. Particularly, the Gb3 accumulated in various cell types of patients with Fabry disease is eliminated by administering Fabrazyme (agalsidase beta) (Eng, C.M. et al. The New England journal of medicine 345, 9-16 (2001)) or Replagal (agalsidase alpha) (Schiffmann, R. et al. Proceedings of the National Academy of Sciences of the United States of America 97, 365-370 (2000)) as alpha-galactosidase. The enzyme administered by intravenous injection enters the cell through the mannose 6-phosphate (M6P) receptor on the plasma membrane and moves to lysosome. Administration of such therapeutic enzymes plays a pivotal role in the treatment of Fabry disease. However, the recombinant enzymes such as Fabrazyme or Replagal are unstable in blood, and there is a possibility that an allergic reaction may be induced by repeated administration (Eng, C. M. et al. The New England journal of medicine 345, 9-16 (2001); Schiffmann, R. et al. Proceedings of the National Academy of Sciences of the United States of America 97, 365-370 (2000); Sakuraba, H. et al. Journal of human genetics 51, 180-188 (2006)).

For the study of Fabry disease and the development of therapeutic agents, it is common to study the role of the Gb3 accumulation in endothelial dysfunction after inducing knockout of GLA in mice. In the GLA knocked-out mouse model, abnormal accumulation of Gb3 was observed in the caveolae of aortic endothelial cells (Shu, L. & Shayman, J. A. The Journal of biological chemistry 282, 20960-20967 (2007)). It has been reported that such abnormal accumulation of Gb3 can cause a decrease in calcium channel function in the GLA knocked-out endothelial cells (Park, S. et al. Cardiovascular research 89, 290-299 (2010)). Nevertheless, since the degree of Gb3 accumulation in the GLA knocked-out mice is somewhat different from that of Fabry disease patients, the GLA knockout mouse model has limitations in that it cannot reproduce various complications induced in Fabry disease patients (Taguchi, Maruyama et al, Biochemical Journal 456, 373-383 (2013)).

On the other hand, stem cells are cells in the pre-differentiation stage into each cell constituting a tissue, and can be obtained from each tissue of the embryo, fetus, and adult. And stem cells refer to cells with autoproliferative ability capable of infinite proliferation in an undifferentiated state and pluripotency, the potential to differentiate into cells of various tissues by specific differentiation stimulation. Stem cells are differentiated into specific cells by stimulation of differentiation (environment), and unlike the differentiated cells in which cell division is stopped, they can produce cells identical to themselves by cell division (self-renewal) and thus have a proliferation (expansion) characteristic. Stem cells can also be differentiated into other cells by different environmental or differentiation stimulations, and thus have the plasticity in differentiation.

Human pluripotent stem cells (hPSCs) including induced pluripotent stem cells (iPSCs) can differentiate into various cell types. Induced pluripotent stem cells (iPSCs) can be differentiated *in vitro* and used to treat diseases without immune rejection, or as a means of understanding and evaluating the early mechanisms of disease (Muotri, A. R. Epilepsy Behav 14 Suppl 1: 81-85 (2009); Marchetto, M. C., B. Winner, et al. Hum Mol Genet 19(R1): R71-76 (2010)).

When the iPSCs derived from patients with various genetic diseases are directly differentiated into disease-related cell types, they exhibit disease-specific phenotypes (Park, I. H. et al. Cell 134, 877-886 (2008); Tiscornia, G. et al. Nature medicine 17, 1570-1576 (2011)). These disease-specific iPSCs can be differentiated into disease-related cell types, and thus can be effectively used to confirm the specific mechanisms of a disease or screen for the therapeutic agents.

Thus, the present inventors recognized the problems of Fabrazyme, which has been used as a therapeutic agent for Fabry disease, and tried to develop a patient-specific therapeutic agent that can overcome the problems. Meanwhile, the present inventors established a stem cell model for studying Fabry disease using iPSCs, and identified that the TSP1 (Thrombospondin1) protein is the cause of vasculopathy of Fabry disease. In addition, the present inventors have completed the present invention by confirming the recovery of cell morphology in vascular endothelial cells derived from a patient with Fabry disease, a decrease in the expression of a TSP1 (Thrombospondin1) gene, a decrease in the expression levels of a TSP1 protein and a phosphorylated-SMAD protein, which are anti-angiogenic factors, and an increase in the expression levels of a KDR (kinase insert domain receptor) protein and an eNOS (endothelial Nitric oxide synthase) protein, which are angiogenic factors.

### SUMMARY OF THE INVENTION

It is an object of the present invention to provide a use of a TSP1 protein inhibitor to prevent or treat Fabry disease.

To achieve the above objects, the present invention provides a pharmaceutical composition comprising a TSP1 (Thrombospondin1) gene expression inhibitor or a TSP1 (Thrombospondin1) protein activity inhibitor as an active ingredient for preventing or treating Fabry disease.

The present invention also provides a health functional food comprising a TSP1 gene expression inhibitor or a TSP1 protein activity inhibitor as an active ingredient for preventing or ameliorating Fabry disease.

The present invention also provides a screening method of a candidate therapeutic agent for Fabry disease, comprising the following steps:
1) treating a test material to a cell line expressing the TSP1 protein;
2) measuring the expression level of the TSP1 protein in the cell line; and
3) selecting a test material that has reduced the expression level of the TSP1 protein compared to the control not treated with the test material.

The present invention also provides a screening method of a candidate therapeutic agent for Fabry disease, comprising the following steps:
1) treating a test material to the TSP1 protein;
2) measuring the activity level of the TSP1 protein; and
3) selecting a test material that has reduced the activity level of the TSP1 protein compared to the control not treated with the test material.

The present invention also provides a protein detection method for providing information necessary for diagnosis of Fabry disease, comprising the following steps:
1) measuring the expression level of the TSP1 protein in the sample derived from a subject; and
2) determining the subject with the increased expression level of the TSP1 protein compared to the control group as a subject at risk of developing Fabry disease.

The present invention also provides a pharmaceutical composition comprising SB-431542 as an active ingredient for preventing or treating Fabry disease.

In addition, the present invention provides a health functional food comprising SB-431542 as an active ingredient for preventing or ameliorating Fabry disease.

### ADVANTAGEOUS EFFECT

In vascular endothelial cells produced by knocking out a TSP1 gene in induced pluripotent stem cells derived from a Fabry disease patient, of the present invention, the recovery of cell morphology, a decrease in the expression of a TSP1 gene, a decrease in the expression levels of a TSP1 protein and a phosphorylated-SMAD protein, which are anti-angiogenic factors, and an increase in the expression levels of a KDR protein and an eNOS protein, which are angiogenic factors, have been confirmed, and thus a TSP1 gene expression inhibitor or a TSP1 protein activity inhibitor can be effectively used in the treatment of Fabry disease.

### BRIEF DESCRIPTION OF THE DRAWINGS

Figure 1 is a diagram showing the expression of the stemness markers OCT4, SOX2, NANOG, SSEA4, Tra-1-81 and Tra-1-60 in induced pluripotent stem cells (FB-iPSCs) prepared from cells of 4 patients with Fabry disease (Fab#1, Fab#2, Fab#3 and Fab#4).
Figure 2 is a diagram showing the karyotype of FB-iPSCs prepared from cells of 4 patients with Fabry disease (Fab#1, Fab#2, Fab#3 and Fab#4).
Figure 3 is a diagram showing the alpha galactosidase (GLA) activity in FB-iPSCs prepared from cells of 4 patients with Fabry disease (Fab#1, Fab#2, Fab#3 and Fab#4).
Figure 4 is a diagram showing the expression level of LC3 protein in FB-iPSCs prepared from cells of 4 patients with Fabry disease (Fab#1, Fab#2, Fab#3 and Fab#4).
Figure 5 is a diagram showing the expression levels of LC3, Beclin 1 and ubiquitin proteins in vascular endothelial cells differentiated from FB-iPSCs derived from 4 patients with Fabry disease (Fab#1, Fab#2, Fab#3 and Fab#4).
Figure 6 is a schematic diagram showing the method of differentiating vascular endothelial cells from FB-iPSCs.
Figure 7 is a diagram showing the proportion of cells expressing CD34 and CD31 simultaneously in vascular endothelial cells differentiated from FB-iPSCs derived from 4 patients with Fabry disease (Fab#1, Fab#2, Fab#3 and Fab#4).
Figure 8 is a diagram showing the distribution of KDR, CD31 and VE-cadherin expressions in vascular endothelial cells differentiated from FB-iPSCs derived from 4 patients with Fabry disease (Fab#1, Fab#2, Fab#3 and Fab#4), and the FACS results of the expression of CD31 and CD105 proteins.
Figure 9 is a diagram confirming the morphology of vascular endothelial cells differentiated from FB-iPSCs derived from a patient with Fabry disease (Fab#1).
Figure 10 is a diagram confirming the tube formation of vascular endothelial cells differentiated from FB-iPSCs derived from 4 patients with Fabry disease (Fab#1, Fab#2, Fab#3 and Fab#4) (FZ: Fabrazyme).
Figure 11 is a diagram showing the GLA activity in vascular endothelial cells differentiated from FB-iPSCs derived from 4 patients with Fabry disease (Fab#1, Fab#2, Fab#3 and Fab#4).
Figure 12 is a diagram confirming the distribution of KDR protein expression in vascular endothelial cells differentiated from FB-iPSCs derived from a patient with Fabry disease (Fab#1).
Figure 13 is a diagram showing the expression levels of TSP1, KDR and eNOS proteins in vascular endothelial cells differentiated from FB-iPSCs derived from 4 patients with Fabry disease (Fab#1, Fab#2, Fab#3 and Fab#4).
Figure 14 is a diagram showing the phosphorylation level of SMAD2 protein in vascular endothelial cells differentiated from FB-iPSCs derived from 4 patients with Fabry disease (Fab#1, Fab#2, Fab#3 and Fab#4) (agal: Fabrazyme).
Figure 15 is a diagram showing the changes in the expression levels of TSP1 protein and angiogenesis-related proteins by the treatment of activin A in vascular endothelial cells differentiated from normal iPSCs (ACTA: activin A).
Figure 16 is a diagram confirming the distribution of TSP1 protein expression in vascular endothelial cells differentiated from FB-iPSCs derived from 4 patients with Fabry disease (Fab#1, Fab#2, Fab#3 and Fab#4).
Figure 17 is a diagram confirming the cell morphology changes by the treatment of LysoGb3 protein in vascular endothelial cells differentiated from normal iPSCs.
Figure 18a is a diagram showing the changes in the expression levels of TSP1, KDR and eNOS genes after the treatment of LysoGb3 protein for 1 day in vascular endothelial cells differentiated from normal iPSCs.
Figure 18b is a diagram showing the changes in the expression levels of TSP1, KDR and eNOS genes after the treatment of LysoGb3 protein for 3 days in vascular endothelial cells differentiated from normal iPSCs.
Figure 18c is a diagram showing the changes in the expression levels of TSP1, KDR and eNOS genes after the treatment of LysoGb3 protein for 5 days in vascular endothelial cells differentiated from normal iPSCs.
Figure 19 is a diagram showing the changes in the expression levels of TSP1, KDR and eNOS proteins, and the changes in the phosphorylation level of AKT protein by the treatment of LysoGb3 protein in vascular endothelial cells differentiated from normal iPSCs.
Figure 20 is a diagram confirming the changes in the distribution of TSP1 protein expression by the treatment of LysoGb3 protein in vascular endothelial cells differentiated from normal iPSCs.
Figure 21a is a diagram confirming the cell morphology changes by the treatment of SB431542 in vascular endothelial cells differentiated from normal iPSCs. (FZ: Fabrazyme, SB: SB431542).
Figure 21b is a diagram confirming the cell morphology changes by the treatment of SB431542 in vascular endothelial cells differentiated from FB-iPSCs derived from a patient with Fabry disease (Fab#1) (FZ: Fabrazyme, SB: SB431542).
Figure 21c is a diagram confirming the cell morphology changes by the treatment of SB431542 in vascular endothelial cells differentiated from FB-iPSCs derived from a patient with Fabry disease (Fab#2) (FZ: Fabrazyme, SB: SB431542).
Figure 21d is a diagram confirming the cell morphology changes by the treatment of SB431542 in vascular endothelial cells differentiated from FB-iPSCs derived from a patient with Fabry disease (Fab#3) (FZ: Fabrazyme, SB: SB431542).
Figure 21e is a diagram confirming the cell morphology changes by the treatment of SB431542 in vascular endothelial cells differentiated from FB-iPSCs derived from a patient with Fabry disease (Fab#4) (FZ: Fabrazyme, SB: SB431542).
Figure 22 is a diagram showing the changes in the expression levels of TSP1, KDR and eNOS proteins by the treatment of SB431542 in vascular endothelial cells differentiated from FB-iPSCs derived from 4 patients with Fabry disease (Fab#1, Fab#2, Fab#3 and Fab#4) (FZ: Fabrazyme, SB: SB431542).
Figure 23a is a diagram confirming the tube formation changes by the treatment of SB431542 in vascular endothelial cells differentiated from normal iPSCs (FZ: Fabrazyme, SB: SB431542).
Figure 23b is a diagram confirming the tube formation changes by the treatment of SB431542 in vascular endothelial cells differentiated from FB-iPSCs derived from a patient with Fabry disease (Fab#1) (FZ: Fabrazyme, SB: SB431542).
Figure 23c is a diagram confirming the tube formation changes by the treatment of SB431542 in vascular endothelial cells differentiated from FB-iPSCs derived from a patient with Fabry disease (Fab#2) (FZ: Fabrazyme, SB: SB431542).
Figure 23d is a diagram confirming the tube formation changes by the treatment of SB431542 in vascular endothelial cells differentiated from FB-iPSCs derived from a patient with Fabry disease (Fab#3) (FZ: Fabrazyme, SB: SB431542).
Figure 23e is a diagram confirming the tube formation changes by the treatment of SB431542 in vascular endothelial cells differentiated from FB-iPSCs derived from a patient with Fabry disease (Fab#4) (FZ: Fabrazyme, SB: SB431542).
Figure 24a is a diagram confirming the cell morphology changes by the treatment of SB431542 or losartan in vascular endothelial cells differentiated from FB-iPSCs derived from a patient with Fabry disease (Fab#1) (FZ: Fabrazyme, SB: SB431542, Los: losartan).
Figure 24b is a diagram confirming the cell morphology changes by the treatment of SB431542 or losartan in vascular endothelial cells differentiated from FB-iPSCs derived from a patient with Fabry disease (Fab#2) (FZ: Fabrazyme, SB: SB431542, Los: losartan) .
Figure 25 is a schematic diagram showing the location of the mutated GLA gene in FB-iPSCs derived from a patient with Fabry disease (Fab#1).
Figure 26 is a schematic diagram showing the method of preparing an isotype control cell line from FB-iPSCs derived from a patient with Fabry disease (Fab#1) using CRISPR/CAS9 system.
Figure 27 is a diagram confirming the correction of GLA gene in the isotype control cell line prepared from FB-iPSCs derived from a patient with Fabry disease (Fab#1).
Figure 28 is a diagram showing the GLA activity in the isotype control cell line prepared from FB-iPSCs derived from a patient with Fabry disease (Fab#1) and vascular endothelial cells differentiated therefrom.
Figure 29 is a diagram showing the expression levels of TSP1 protein and angiogenesis-related protein, and the phosphorylation level of SMAD protein in vascular endothelial cells differentiated from the isotype control cell line prepared from FB-iPSCs derived from a patient with Fabry disease (Fab#1) (agal: Fabrazyme).
Figure 30 is a diagram confirming the distribution of TSP1 and VE-cadherin expression in vascular endothelial cells differentiated from the isotype control cell line prepared from FB-iPSCs derived from a patient with Fabry disease (Fab#1) (agal: Fabrazyme).
Figure 31 is a diagram confirming the tube formation of vascular endothelial cells differentiated from the isotype control cell line prepared from FB-iPSCs derived from a patient with Fabry disease (Fab#1) (agal: Fabrazyme).
Figure 32 is a schematic diagram showing the method of preparing a TSP1 gene knockout cell line from FB-iPSCs derived from a patient with Fabry disease (Fab#1) using CRISPR/CAS9 system.
Figure 33 is a diagram confirming the knockout of the TSP1 gene in the TSP1 gene knockout cell line prepared from FB-iPSCs derived from a patient with Fabry disease (Fab#1) by the sequencing method.
Figure 34 is a diagram confirming the morphology of vascular endothelial cells differentiated from FB-iPSCs derived from a patient with Fabry disease (Fab#1) in which the TSP1 gene was knocked out (agal: Fabrazyme).
Figure 35 is a diagram showing the expression level of TSP1 gene in vascular endothelial cells differentiated from FB-iPSCs derived from a patient with Fabry disease (Fab#1) in which the TSP1 gene was knocked out.
Figure 36 is a diagram showing the expression levels of TSP1, KDR and eNOS proteins, and the phosphorylation level of SMAD protein in vascular endothelial cells differentiated from FB-iPSCs derived from a patient with Fabry disease (Fab#1) in which the TSP1 gene was knocked out (agal: Fabrazyme).
Figure 37 is a diagram confirming the tube formation of vascular endothelial cells differentiated from FB-iPSCs derived from a patient with Fabry disease (Fab#1) in which the TSP1 gene was knocked out (agal: Fabrazyme).

### DESCRIPTION OF THE PREFERRED EMBODIMENTS

Hereinafter, the present invention is described in detail.

The present invention provides a pharmaceutical composition comprising a TSP1 gene expression inhibitor or a TSP1 protein activity inhibitor as an active ingredient for preventing or treating Fabry disease.

The TSP1 gene can include all polynucleotides composed of any sequence known in the art, and can include a variant having one or more substitutions, insertions, deletions, and combinations thereof that do not reduce the activity. The variant can have 80%, 90%, 95%, 98% or more homology with the TSP1 gene sequence of the present invention. In an embodiment of the present invention, the TSP1 gene can be a polynucleotide composed of the nucleotide sequence represented by SEQ. ID. NO: 1. The TSP1 gene expression inhibitor can include any material known in the art as long as it suppresses mRNA of the gene encoding TSP1 protein, and can be prepared by various methods such as chemical synthesis or in vitro transcription synthesis. The expression inhibitor can include any one or more selected from the group consisting of antisense nucleotide, siRNA (small interfering RNA), shRNA (short hairpin RNA) and ribozyme complementarily binding to the polynucleotide constituting TSP1 gene.

The antisense nucleotide, as defined in the Watson-Crick base pair, refers to a sequence that interferes with the flow of genetic information from DNA to protein by binding (hybridizing) to the complementary nucleotide sequence of DNA, immature-mRNA or mature mRNA. In addition, since the antisense nucleotide is a long chain of monomer units, the antisense nucleotide for target RNA sequence can be easily synthesized.

The said siRNA refers to a short double-stranded RNA capable of inducing RNA interference through cleavage of specific mRNA. In addition, the siRNA is not limited to the region in which the double-stranded RNA is completely paired, and can include the region in which the strand is not paired due to mismatch (the corresponding nucleotide is not complementary) or bulge (no nucleotide corresponding to one chain). The siRNA terminal structure can have either a blunt end or a protruding end, as long as the expression of the target gene can be suppressed by the RNA interference effect, and the adhesive terminal structure can be both a 3' end protruding structure and a 5' end protruding structure.

The said shRNA refers to a sequence of RNA that makes a strong hairpin turn, which can be used to silence gene expression through RNA interference. In addition, the shRNA can be delivered to cells using a vector for cell introduction, and such vector is always delivered to daughter cells, so that gene silencing can be inherited. The shRNA hairpin structure is degraded into siRNA by an intracellular mechanism and bound to an RNA-induced silencing complex, and the complex binds to mRNA corresponding to the siRNA to degrade it.

The said ribozyme refers to an enzyme RNA molecule capable of catalyzing specific RNA cleavage. Endonucleolytic cleavage can be induced by the specific hybridization of the molecular sequence of ribozyme and the complementary target RNA. The ribozyme can include the known sequences responsible for the cleavage of one or more sequences complementary to the target RNA or the functionally equivalent sequences. In addition, the ribozyme can be a hammer-head ribozyme or a Cech type ribozyme, the endoribonuclease RNA, and can be formed by a modified oligonucleotide to improve safety and targeting. Meanwhile, the ribozyme can be distributed in cells expressing a target gene in vivo. DNA constructs encoding ribozyme under the control of a strong constitutive polymerase III or polymerase II promoter so that the transfected cells can destroy the endogenous target messenger and produce a sufficient amount of ribozyme to inhibit translation can be used. Since the ribozyme has a catalytic activity, it may have to be maintained at a low concentration in cells unlike other antisense molecules.

The said TSP1 protein can include a polypeptide composed of any sequence known in the art. The polypeptide can be a variant of amino acids having a different sequence by deletion, insertion, substitution or a combination thereof of amino acid residues, within a range that does not affect the function of the protein. Amino acid exchange in proteins or peptides that do not alter the activity of the molecule as a whole is known in the art. In some cases, it can be modified by phosphorylation, sulfation, acrylation, glycosylation, methylation, farnesylation, etc. The variant can have homology with at least 80%, 90%, or 95% with the TSP1 protein of the present invention. In an embodiment of the present invention, the TSP1 protein can be a polypeptide composed of the amino acid sequence represented by SEQ. ID. NO: 2.

The TSP1 protein activity inhibitor can include any one or more selected from the group consisting of compounds, peptides, peptide mimetics, matrix analogs, aptamers, and antibodies that complementarily bind to the polypeptide constituting the TSP1 protein.

The peptide and peptide mimetics can inhibit the TSP1 protein activity by suppressing the binding of the TSP1 protein to other proteins. The peptide mimetics can be a peptide or a non-peptide, and can be composed of the amino acids bound by a non-peptide bond such as a psi bond. Non-hydrolyzable peptide mimetics can be prepared using β-turn dipeptide core, keto-methylene pseudopeptides, azepine, benzodiazepine, β-amino alcohol or substituted gammalactam ring as a main residue.

The said aptamer refers to a single-stranded nucleic acid (DNA, RNA or modified nucleic acid) that has a stable tertiary structure by itself and can bind to a target molecule with high affinity and specificity. Since the aptamer can bind to organic matters, peptides, membrane proteins, etc., and block the function thereof, it can be regarded as a kind of a chemical antibody that replaces a single antibody. In addition, the aptamer can be obtained by separating oligomers that bind to specific chemical molecules or biological molecules with high affinity and selectivity using an oligonucleotide library called SELEX (systematic evolution of ligands by exponential enrichment) .

The said antibody can be a monoclonal antibody, a polyclonal antibody, or a recombinant antibody. Antibodies against a specific protein can be easily prepared using the techniques well known in the art if the sequence of the protein is informed. Particularly, antibodies can be prepared using a hybridoma method or a phage antibody library technique. In general, hybridoma cells secreting monoclonal antibodies can be prepared by fusing immune cells isolated from immunologically suitable host animals such as mice injected with antigen proteins and cancer cell lines. The fusion of these two groups of cells can be performed using polyethylene glycol or the like, and the antibody-producing cells can be proliferated by standard culture methods. After a homogeneous cell population is obtained by subcloning using a limiting dilution method, hybridoma cells capable of producing antigen-specific antibodies can be prepared by mass-culture in vitro or in vivo. The antibodies prepared by the above methods can be separated and purified using the methods such as gel electrophoresis, dialysis, salt precipitation, ion exchange chromatography, and affinity chromatography, etc.

The polyclonal antibody can be prepared by injecting a biomarker protein or a fragment thereof as an immunogen into an external host. The external host can be mammals such as mice, rats, sheep, and rabbits. When the immunogen is injected by intramuscular, intraperitoneal or subcutaneous injection, it can be administered with an adjuvant for increasing antigenicity. Thereafter, blood is regularly collected from the external host to obtain serum showing improved titer and specificity to the antigen, and the antibody can be isolated and purified therefrom.

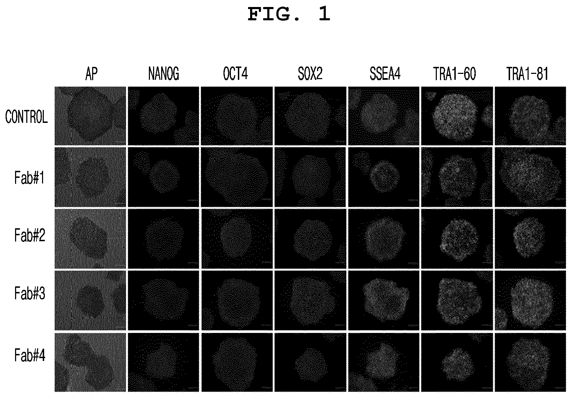

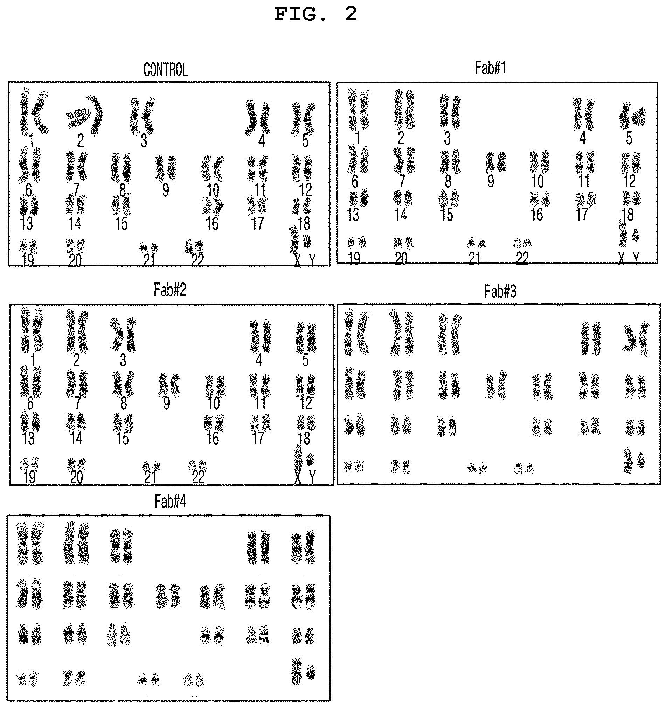

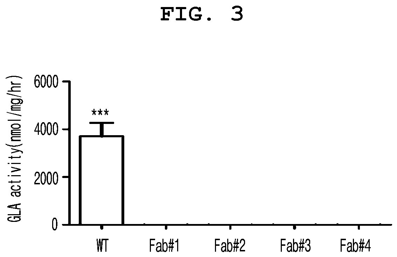

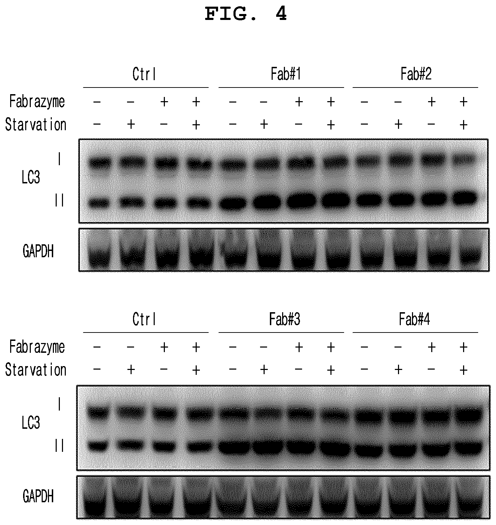

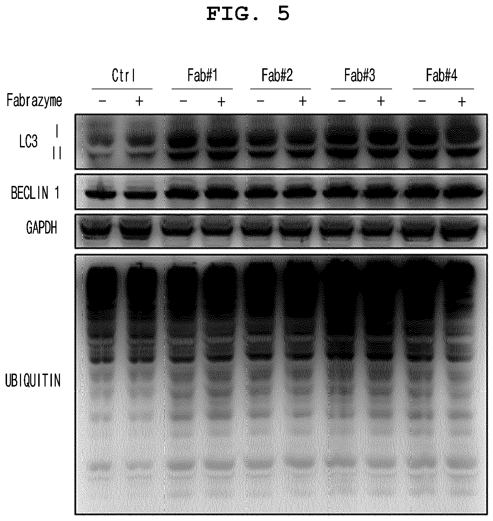

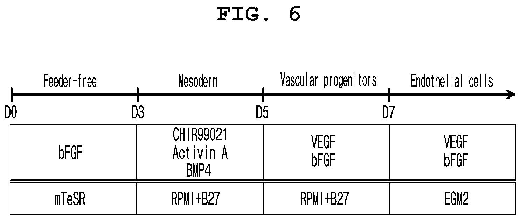

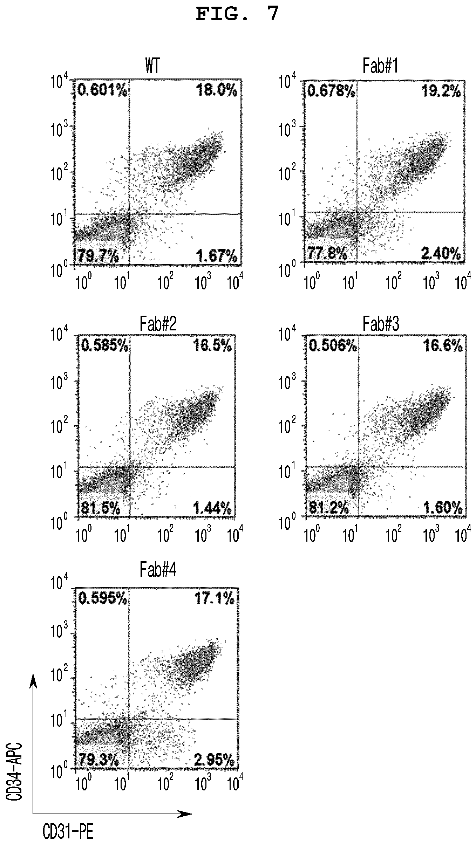

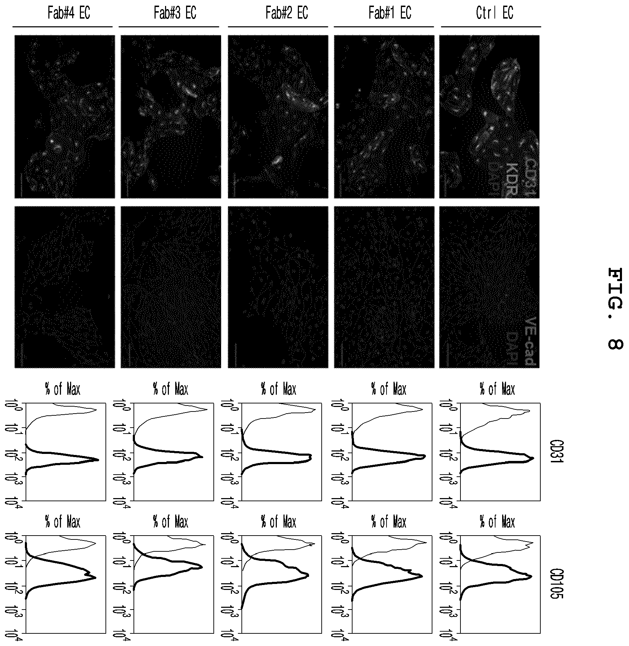

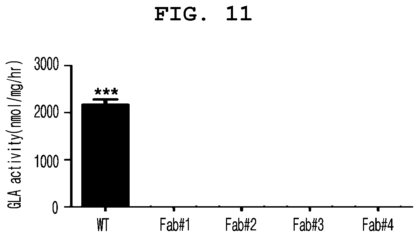

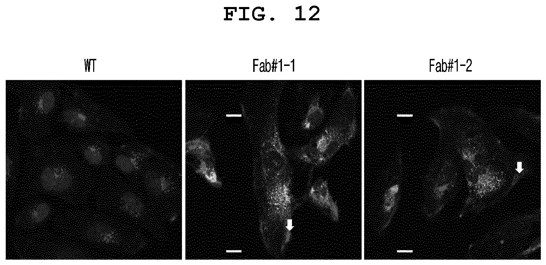

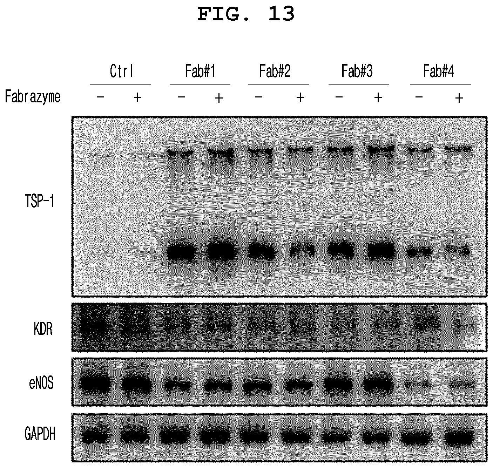

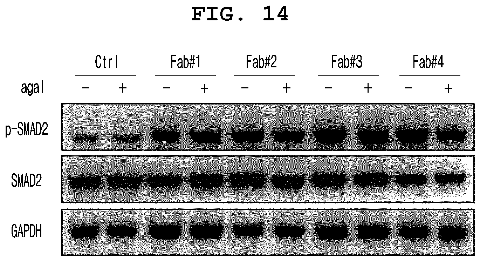

In a preferred embodiment of the present invention, the present inventors prepared induced pluripotent stem cells (FB-iPSCs) using cells of patients with Fabry disease, and confirmed the characteristics of the stem cells (Figures 1 and 2), the decrease of the GLA activity (Figure 3), and the increase of the expression levels of lysosome-related proteins (Figures 4 and 5). The present inventors also prepared vascular endothelial cells differentiated from FB-iPSCs (Figures 6 and 7), and confirmed the characteristics of the vascular endothelial cells (Figures 8 ∼ 10), the decrease of the GLA activity (Figure 11), the decrease of the expression levels of KDR protein and eNOS protein (Figures 12 and 13), and the increase of the expression level of TSP1 protein (Figures 14 ~ 16).

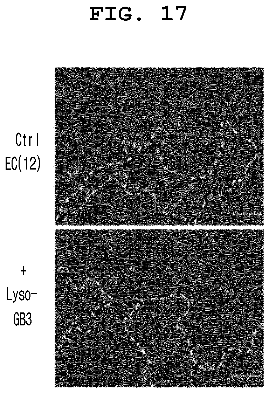

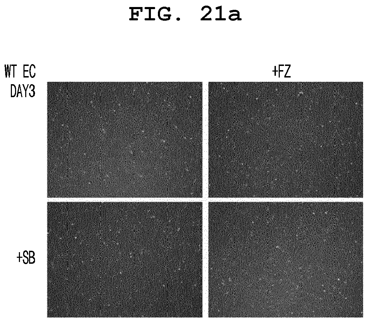

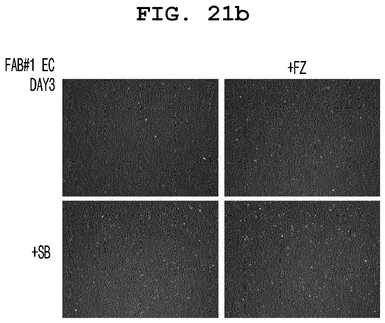

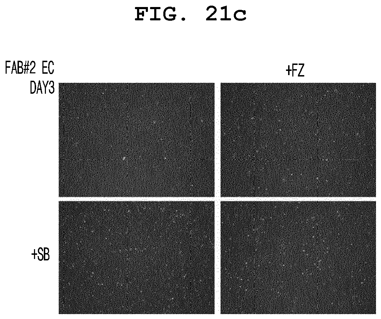

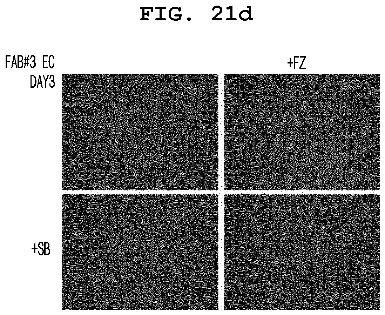

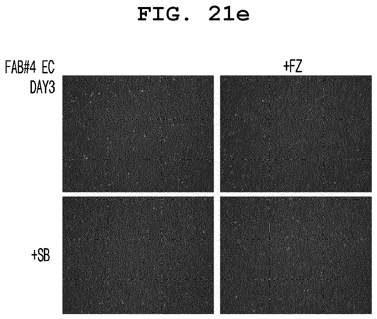

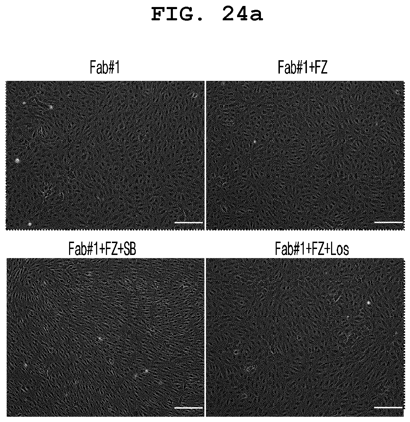

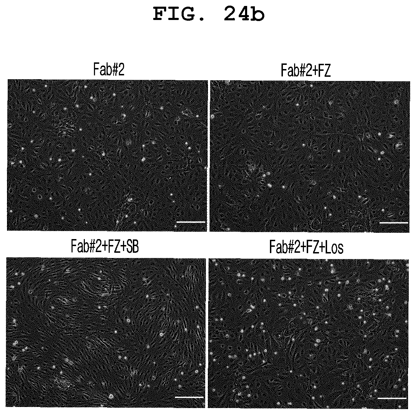

The present inventors also confirmed that vasculopathy was induced in vascular endothelial cells differentiated from FB-iPSCs by Gb3 protein (Figures 17 ~ 20), and SB431542, a TGF-β inhibitor, had an effect on the treatment of vasculopathy (Figures 21 ~ 23). However, the therapeutic effect of losartan, another TGF-β inhibitor, could not be confirmed (Figure 24).

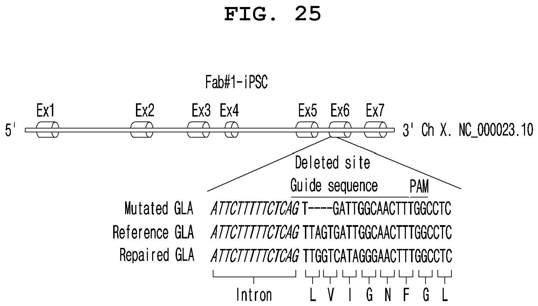

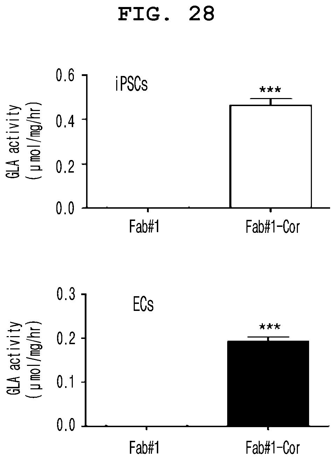

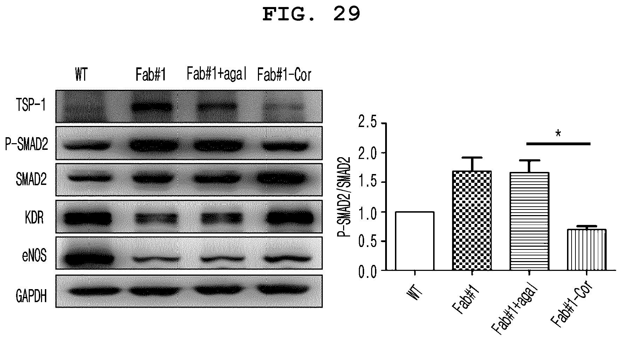

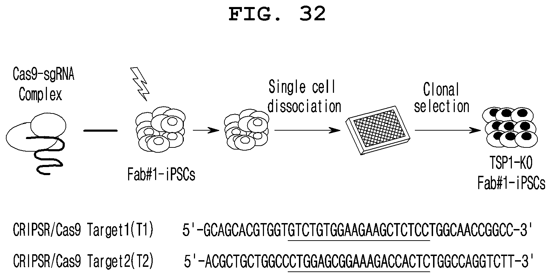

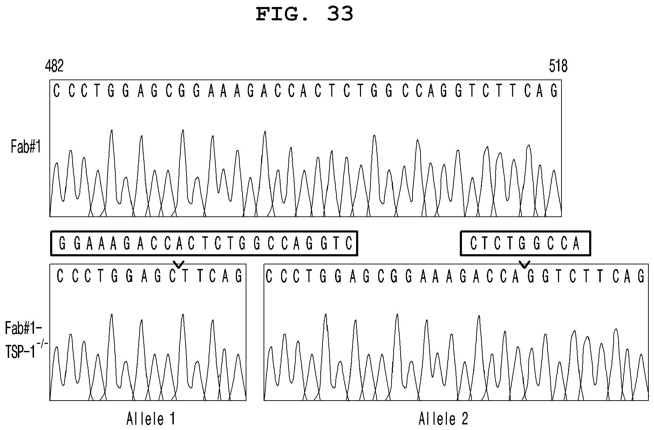

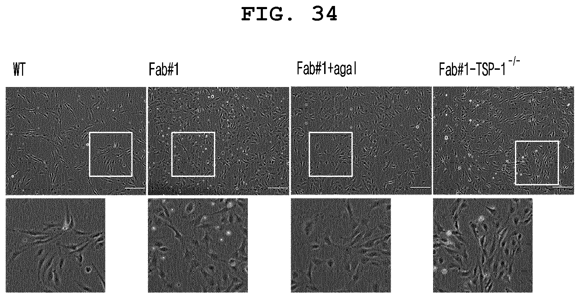

In addition, the present inventors prepared an isotype control cell line of FB-iPSCs (Figures 25 ∼ 27) and vascular endothelial cells differentiated therefrom, and confirmed the recovery of the GLA activity (Figure 28) and vasculopathy (Figures 29 ∼ 31). The present inventors also prepared a FB-iPSC cell line in which the TSP1 gene was knocked out (Figures 32 and 33) and confirmed the recovery of the vasculopathy (Figures 34 ~ 37).

Therefore, the TSP1 gene expression inhibitor or the TSP1 protein activity inhibitor of the present invention can be effectively used for the prevention or treatment of Fabry disease.

The pharmaceutical composition can contain the TSP1 gene expression inhibitor or the TSP1 protein activity inhibitor of the present invention as an active ingredient at the concentration of 10 ~ 95 weight% by the total weight of the composition. In addition, the pharmaceutical composition of the present invention can contain, in addition to the active ingredient, one or more effective ingredients having the same or similar function to the active ingredient.

The pharmaceutical composition of the present invention can contain carriers, diluents, excipients, or combinations of at least two of those generally used in biological preparations. The pharmaceutically acceptable carrier can be any carrier that is able to deliver the composition of the present invention in a living body without limitation, which is exemplified by the compounds described in Merck Index, 13th ed., Merck & Co. Inc., such as saline, sterilized water, Ringer's solution, buffered saline, dextrose solution, maltodextrin solution, glycerol, ethanol, and a mixture comprising one or more of those components. If necessary, a general additive such as antioxidant, buffer, and bacteriostatic agent can be additionally added.

When the composition is formulated, it is prepared using generally used diluents or excipients such as fillers, extenders, binders, wetting agents, disintegrating agents and surfactants.

The composition of the present invention can be formulated as an oral or parenteral formulation. Solid formulations for oral administration are tablets, pills, powders, granules and capsules. These solid formulations are prepared by mixing one or more compositions with one or more suitable excipients such as starch, calcium carbonate, sucrose, lactose and gelatin, etc. In addition, lubricants such as magnesium stearate and talc can be added thereto. Liquid formulations for oral administrations are suspensions, solutions, emulsions or syrups, and the above-mentioned formulations can contain various excipients such as wetting agents, sweeteners, aromatics and preservatives.

Formulations for parenteral administration are sterilized aqueous solutions, water-insoluble excipients, suspensions, and emulsions. Water insoluble excipients and suspensions can contain propylene glycol, polyethylene glycol, vegetable oil like olive oil, injectable ester like ethylolate, etc.

The composition of the present invention can be administered by orally or parenterally and the parenteral administration can be selected from the group consisting of skin external application or intraperitoneal injection, intrarectal injection, subcutaneous injection, intravenous injection, intramuscular injection, and intrathoracic injection.

The composition can be administered by the pharmaceutically effective amount. The effective amount can be determined according to the type of disease, the severity, the activity of the drug, the patient's sensitivity to the drug, the time of administration, the route of administration, the duration of treatment, the drugs being used simultaneously, and the like. The composition of the present invention can be administered alone or in combination with other therapeutic agents. In combination administration, the administration can be sequential or simultaneous. However, for the desired effect, the amount of the active ingredient included in the pharmaceutical composition according to the present invention can be 0.001 ~ 10,000 **mg/kg,** specifically 0.1 ~ 5 g/kg. The administration frequency can be once a day or a few times a day.

The present invention also provides a health functional food comprising a TSP1 gene expression inhibitor or a TSP1 protein activity inhibitor as an active ingredient for preventing or ameliorating Fabry disease.

The TSP1 gene and the TSP1 gene expression inhibitor can have the characteristics as described above. For example, the TSP1 gene can be a polynucleotide composed of the nucleotide sequence represented by SEQ. ID. NO: 1. In addition, the expression inhibitor can include any one or more selected from the group consisting of antisense nucleotide, siRNA, shRNA and ribozyme complementarily binding to the polynucleotide constituting TSP1 gene.

The TSP1 protein and the TSP1 protein activity inhibitor can have the characteristics as described above. For example, the TSP1 protein can be a polypeptide composed of the amino acid sequence represented by SEQ. ID. NO: 2. In addition, the activity inhibitor can include any one or more selected from the group consisting of compounds, peptides, peptide mimetics, matrix analogs, aptamers, and antibodies that complementarily bind to the polypeptide constituting the TSP1 protein.

In a preferred embodiment of the present invention, the present inventors prepared induced pluripotent stem cells (FB-iPSCs) using cells of patients with Fabry disease, and confirmed the characteristics of the stem cells (Figures 1 and 2), the decrease of the alpha-galactosidase (GLA) activity (Figure 3), and the increase of the expression levels of lysosome-related proteins (Figures 4 and 5). The present inventors also prepared vascular endothelial cells differentiated from FB-iPSCs (Figures 6 and 7), and confirmed the characteristics of the vascular endothelial cells (Figures 8 ~ 10), the decrease of the GLA activity (Figure 11), the decrease of the expression levels of KDR protein and eNOS protein (Figures 12 and 13), and the increase of the expression level of TSP1 protein (Figures 14 ~ 16).

The present inventors also confirmed that vasculopathy was induced in vascular endothelial cells differentiated from FB-iPSCs by Gb3 protein (Figures 17 ~ 20), and SB431542, a TGF-β inhibitor, had an effect on the treatment of vasculopathy (Figures 21 ~ 23). However, the therapeutic effect of losartan, another TGF-β inhibitor, could not be confirmed (Figure 24).

In addition, the present inventors prepared an isotype control cell line of FB-iPSCs (Figures 25 ∼ 27) and vascular endothelial cells differentiated therefrom, and confirmed the recovery of the GLA activity (Figure 28) and vasculopathy (Figures 29 ∼ 31). The present inventors also prepared a FB-iPSC cell line in which the TSP1 gene was knocked out (Figures 32 and 33) and confirmed the recovery of the vasculopathy (Figures 34 ~ 37).

Therefore, the TSP1 gene expression inhibitor or the TSP1 protein activity inhibitor of the present invention can be effectively used for the prevention or amelioration of Fabry disease.

The TSP1 gene expression inhibitor or the TSP1 protein activity inhibitor of the present invention can be used as a food additive. In that case, the TSP1 gene expression inhibitor or the TSP1 protein activity inhibitor of the present invention can be added as it is or as mixed with other food components according to the conventional method. The amount of the active ingredient can be regulated according to the purpose of use. In general, the active ingredient of the present invention is preferably added to the health functional food by 0.01 ~ 90 weight part for the total weight of the food.

In addition, there is no particular limitation on the form and type of the health functional food. The form of the health functional food to which the above substances can be added may be tablets, capsules, powders, granules, liquids and pills.

The health functional food of the present invention can additionally include various flavors or natural carbohydrates, etc, like other conventional health foods. The natural carbohydrates above can be one of monosaccharides such as glucose and fructose, disaccharides such as maltose and sucrose, polysaccharides such as dextrin and cyclodextrin, and glucose alcohols such as xilytole, sorbitol and erythritol. Besides, natural sweetening agents such as thaumatin and stevia extract, and synthetic sweetening agents such as saccharin and aspartame can be included as a sweetening agent.

In addition to the ingredients mentioned above, the health functional food of the present invention can include in a variety of nutrients, vitamins, minerals, flavors, coloring agents, pectic acid and its salts, alginic acid and its salts, organic acid, protective colloidal viscosifiers, pH regulators, stabilizers, antiseptics, glycerin, alcohols, etc. All the mentioned ingredients can be added singly or together. The ratio of those ingredients can be selected in the range of 0.01 ~ 0.1 weight part per 100 weight part of the composition of the present invention.

The present invention also provides a screening method of a candidate therapeutic agent for Fabry disease using a TSP1 protein.

The TSP1 protein can have the characteristics as described above. For example, the TSP1 protein can be a polypeptide composed of the amino acid sequence represented by SEQ. ID. NO: 2.

Specifically, the method can be composed of the following steps:
1) treating a test material to a cell line expressing the TSP1 protein;
2) measuring the expression level of the TSP1 protein in the cell line; and
3) selecting a test material that has reduced the expression level of the TSP1 protein compared to the control not treated with the test material.

The test material of step 1) can be one or more substances selected from the group consisting of peptides, proteins, non-peptidic compounds, active compounds, fermentation products, cell extracts, plant extracts, animal tissue extracts and plasma.

The protein expression level of step 2) can be measured by any one or more methods selected from the group consisting of enzyme-linked immunosorbent assay (ELISA), radioimmunoassay, sandwich enzyme immunoassay, western blot, immunoprecipitation, immunohistochemistry, fluoroimmunoassay and flow cytometry (FACS).

Another method can be composed of the following steps:
1) treating a test material to the TSP1 protein;
2) measuring the activity level of the TSP1 protein; and
3) selecting a test material that has reduced the activity level of the TSP1 protein compared to the control not treated with the test material.

The test material of step 1) can be one or more substances selected from the group consisting of peptides, proteins, non-peptidic compounds, active compounds, fermentation products, cell extracts, plant extracts, animal tissue extracts and plasma.

The protein activity level of step 2) can be measured by any one or more methods selected from the group consisting of enzyme immunoassay, fluoroimmunoassay, SDS-PAGE, mass spectrometry and protein chip.

In a preferred embodiment of the present invention, the present inventors confirmed the increase of the expression level of TSP1 protein in vascular endothelial cells differentiated from FB-iPSCs derived from patients with Fabry disease (Figures 14 ~ 16), and the recovery of vasculopathy in vascular endothelial cells differentiated from a FB-iPSC cell line in which the TSP1 gene was knocked out (Figures 34 ~ 37). Therefore, the method of measuring the level of the expression or the activity of the TSP1 protein can be effectively used for screening a candidate therapeutic agent for Fabry disease.

The present invention also provides a protein detection method for providing information necessary for diagnosis of Fabry disease using a TSP1 protein.

The TSP1 protein can have the characteristics as described above. For example, the TSP1 protein can be a polypeptide composed of the amino acid sequence represented by SEQ. ID. NO: 2.

Specifically, the method can be composed of the following steps:
1) measuring the expression level of the TSP1 protein in the sample derived from a subject; and
2) determining the subject with the increased expression level of the TSP1 protein compared to the control group as a subject at risk of developing Fabry disease.

In a preferred embodiment of the present invention, the present inventors confirmed the increase of the expression level of TSP1 protein in vascular endothelial cells differentiated from FB-iPSCs derived from patients with Fabry disease (Figures 14 ~ 16), and the recovery of vasculopathy in vascular endothelial cells differentiated from a FB-iPSC cell line in which the TSP1 gene was knocked out (Figures 34 ~ 37). Therefore, the method of measuring the level of the expression of the TSP1 protein can be effectively used for providing information necessary for diagnosis of Fabry disease.

The present invention also provides a pharmaceutical composition comprising SB-431542, a TGF-β signaling pathway inhibitor, as an active ingredient for preventing or treating Fabry disease.

The "TGF (transforming growth factor) beta signaling pathway inhibitor" means a substance that inhibits signaling mediated by TGF beta. The TGF beta is a protein that regulates various physiological processes such as cell proliferation, differentiation, apoptosis, migration, extracellular matrix (ECM) production, angiogenesis, and development in vivo.

The TGF beta signaling pathway inhibitor can be SB-431542, but any substance capable of inhibiting TGF signaling can be used without limitation. In the present invention, SB431542 was used as the TGF beta signaling pathway inhibitor, and it can be represented by formula 1 below.

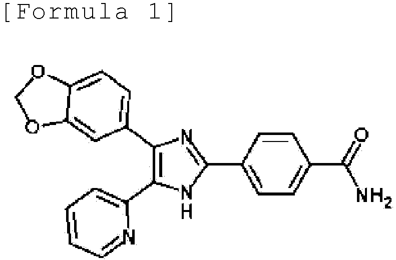

The said SB-431542 can reduce the expression of TSP1 protein in vascular endothelial cells of patients with Fabry disease, and can increase the expressions of KDR (kinase insert domain receptor) and eNOS (endothelial Nitric oxide synthase) proteins.

In a preferred embodiment of the present invention, the present inventors confirmed that vasculopathy was induced in vascular endothelial cells differentiated from FB-iPSCs by Gb3 protein (Figures 17 ~ 20), and SB431542, a TGF-β inhibitor, had an effect on the treatment of vasculopathy (Figures 21 ~ 23). Therefore, the SB-431542 compound of the present invention can be effectively used for the prevention or treatment of Fabry disease.

The pharmaceutical composition can have the characteristics as described above.

In addition, the present invention provides a health functional food comprising SB-431542, a TGF-β signaling pathway inhibitor, as an active ingredient for preventing or ameliorating Fabry disease.

The TGF-β signaling pathway inhibitor or SB-431542 can have the characteristics as described above. For example, the SB-431542 can have the structure of formula 1 below, can reduce the expression of TSP1 protein in vascular endothelial cells of patients with Fabry disease, and can increase the expressions of KDR and eNOS proteins. 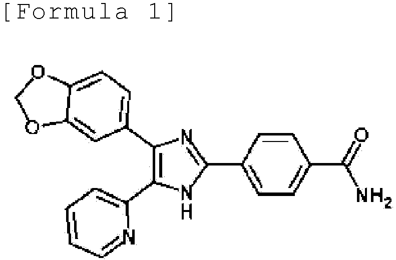 In a preferred embodiment of the present invention, the present inventors confirmed that vasculopathy was induced in vascular endothelial cells differentiated from FB-iPSCs by Gb3 protein (Figures 17 ~ 20), and SB431542, a TGF-β inhibitor, had an effect on the treatment of vasculopathy (Figures 21 ~ 23). Therefore, the SB-431542 compound of the present invention can be effectively used for the prevention or amelioration of Fabry disease.

The health functional food can have the characteristics as described above.

Hereinafter, the present invention will be described in detail by the following examples.

However, the following examples are only for illustrating the present invention, and the contents of the present invention are not limited thereto.

### Example 1: Preparation of induced pluripotent stem cells (iPSCs) derived from patients with Fabry disease

For the study of Fabry disease, fibroblasts were isolated from patients with Fabry disease, and iPSCs were induced in the following manner.

Particularly, with the consent of patients with Fabry disease at Asan Medical Center (Seoul, Korea), the patients were subjected to local anesthesia, and then the dermal tissue was obtained by performing skin tissue biopsy using punch biopsy. Clinical information of 4 patients with Fabry disease is shown in Table 1 below. All the experiments were conducted under the review of Institutional Review Board of the hospital.

**[Table 1]**

| Patient No. | 1 | | 2 | | 3 | | 4 | |
|---|---|---|---|---|---|---|---|---|
| Gender | Male | | Male | | Male | | Male | |
| Age | 37 | | 28 | | 50 | | 28 | |
| Diagnosis | 18 | | 24 | | 32 | | 20 | |
| Phenotype | Classical | | Classical | | Classical | | Classical | |
| GLA activity | 0.0% | | 0.6% | | 0.0% | | 0.6% | |
| GLA mutation c. | c.803_806 del | | c.658C>T | | c.334C>T | | c.1045T>C | |
| GLA mutation p. | p.L268fs* 1 | | p.Arg220* | | p.Arg112C ys | | p.Trp349A rg | |
| ERT duration | 0 | 12 years | 0 | 3 years | 0 | 11 years | 0 | 7 years |
| Serum GL3 (3.9-9.9 ug/ml) | 18 | 4.8 | 16 | 8.3 | 10 | 5.5 | 12 | 5.6 |
| Angiokeratoma | + | + | + | + | + | + | + | + |
| Cornea verticil lata | + | + | + | + | + | + | + | + |
| Sensorineural hearing loss | + | + | - | + | + | + | - | - |
| EKG abnormality | WPW | WPW | SB | WPW | SB | SB | WPW | WPW |
| Cardiac hypertrophy | - | - | - | - | + | + | - | - |
| LV mass (g/m²) | NA | 89 | 89 | 98 | 126 | 153 | 94 | 105 |
| Anhidrosis/Hypohydrosis | + | + | + | + | + | + | + | + |
| Acroparesthesia | + | + | + | + | - | - | + | + |
| Proteinuria (mg/day/m²) | 11 | 106 | 0.0 3 | 73 | 109 | 98 | 72 | 35 |
| GFR (ml/min/1.73m²) | 141 | 139 | 125 | 122 | 58 | 46 | 123 | 122 |

Fibroblasts were isolated from the obtained dermal tissue and cultured in DMEM supplemented with 10% fetal bovine serum (FBS), 1% penicillin-streptomycin and 1% non-essential amino acid. Then, FB-iPSC development was induced from the fibroblasts using a technique known in the art for ectopic expression by transduction of retrovirus expressing the pluripotent markers OCT4, SOX2, KLF4 and C-MYC (Takahashi, K et al, Cell 131(5) : 861-872, 2007). The cells were cultured for 3 days, subcultured using mouse fetal fibroblasts as culture feeder cells, and cultured in DMEM/F12 medium supplemented with 20% KOSR (knockout serum replacement), 50 units/ml of penicillin-streptomycin, 0.1 mM non-essential amino acid, 1 mM L-glutamine, 0.1 mM β-mercaptoethanol and 10 ng/ml of basic fibroblast growth factor for a long time. Thereafter, a colony having the morphological characteristics similar to that of stem cells was isolated to construct an FB-iPSC cell line.

### Experimental Example 1: Confirmation of stem cell characteristics of iPSCs derived from patients with Fabry disease

### <1-1> Confirmation of pluripotency of FB-IPSCs

In order to confirm whether the undifferentiated FB-iPSCs exhibit pluripotency, the expression of stemness markers in FB-iPSCs was confirmed using immunostaining.

Particularly, the FB-iPSCs prepared in Example 1 were fixed using a formaldehyde solution, and 0.1% triton X-100 was treated thereto to make the cell membrane permeable. Thereafter, the treated cells were washed three times with PBST (PBS containing Tween-20), to which and 4% normal donkey serum was added, followed by blocking at room temperature for 1 hour. Then, as the primary antibody, anti-OCT4 antibody (1:300, R&D Systems, USA), anti-SOX2 antibody (BD Transduction Laboratories, USA), anti-NANOG antibody (1:300, Cell signaling technology, USA), anti-SSEA-4 antibody (1:300, R&D Systems, USA), anti-TRA-1-81 antibody (1:300, Millipore, USA) or anti-TRA-1-60 antibody (1:300, Millipore, USA) was treated thereto and reacted at 4°C, followed by washing three times with PBST. After washing, Alexa Fluor 488 or Alexa Fluor 594-conjugated secondary antibody (Invitrogen, USA) was treated thereto and reacted at room temperature for 1 hour, followed by washing 6 times with PBST. At this time, the nuclei were fluorescently stained with DAPI (4',6-diamidino-2-phenylindole) at the time of the last washing. Then, the expressions of OCT4, SOX2, NANOG, SSEA4, TRA-1-81 and TRA-1-60 proteins were confirmed by observing the stained FB-iPSCs under a fluorescence microscope (Olympus, Japan) or a Zeiss LSM 510 confocal microscope (Carl Zeiss, Germany).

As a result, as shown in Figure 1, the stemness markers OCT4, SOX2, NANOG, SSEA4, Tra-1-81 and Tra-1-60 proteins were expressed in FB-iPSCs at the levels in normal cells. From the above results, it was confirmed that the FB-iPSCs had the same pluripotency as embryonic stem cells.

### <1-2> Confirmation of karyotype of FB-IPSCs

Karyotyping of the FB-iPSCs prepared in Example 1 was requested to GenDix (Korea), and it was confirmed that the FB-iPSCs prepared in Example 1 had a normal karyotype.

As shown in Figure 2, the FB-iPSCs had a normal karyotype of 46, XY.

### Experimental Example 2: Confirmation of decreased activity of alpha-galactosidase (a-galactosidase, GLA) in FB-iPSCs

In patients with Fabry disease, GLA activity should appear close to zero, so the GLA activity in FB-iPSCs was confirmed by the following method.

Particularly, 200 µl of GLA assay buffer (pH 4.6) containing 100 mM sodium citrate and 200 mM sodium phosphate dibasic was added to cell pellet of the FB-iPSCs prepared in Example 1, followed by sonication with a program of 1-second crushing and 1-second rest for a total of 10 seconds. Thereafter, a supernatant of the cell lysate was obtained by centrifugation at 1300 rpm for 30 minutes at 4°C. Then, a mixture for GLA analysis was prepared by mixing 30 µl of 200 mM N-acetyl-D-galactosamine (GalNAC), 6 µl of 50 mM 4-MU-α-D-galactopyranoside (4MUaGal), 14 µl of GLA assay buffer, and 10 µl of the obtained cell lysate supernatant. The prepared mixture was added to a 96 well plate, followed by incubation at 37°C for 1 hour. Upon completion of the culture, the reaction was terminated by adding GLA stop buffer containing 200 mM glycine (pH 10.32) to each well, and fluorescence was confirmed at wavelengths of 355 and 460 nm using Victor plate reader (Perkin Elmer, USA).

As a result, as shown in Figure 3, the GLA activity was not shown in FB-iPSCs, unlike in the normal control group.

### Experimental Example 3: Confirmation of lysosome-related protein expression level in patients with Fabry disease

Since Fabry disease is classified as a lysosomal storage disease, the expression levels of lysosome-related proteins in each of FB-iPSCs and Fabry disease patient samples were confirmed by the following method.

### <3-1> Confirmation of LC3 (autophagy-related protein light chain 3) protein expression level in FB-iPSCs

The expression level of the lysosome-related protein LC3 in FB-iPSCs was confirmed by Western blotting.

Particularly, the FB-iPSCs prepared in Example 1 were treated with Fabrazyme, a therapeutic agent for Fabry disease, and starvation media in various combinations. Pro-Prep solution (Intron Biotechnology, Korea) was added to each sample and reacted at 4°C, followed by centrifugation at 1300 rpm for 10 minutes to obtain a supernatant. The obtained supernatant was electrophoresed on acrylamide gel and transferred to a nitrocellulose membrane, followed by blocking with 4% skim milk. Then, anti-LC3 antibody (1:1000, Santacruz, USA) was treated thereto and reacted at 4°C for 24 hours, followed by washing three times with TBS containing tween-20. The protein expression level was confirmed by the conventional Western blotting. The expression level of each protein was corrected using GAPDH as a control.

As a result, as shown in Figure 4, the expression level of LC3 protein was higher in FB-iPSCs than in normal cells regardless of the Fabrazyme treatment.

### <3-2> Confirmation of expression levels of LC3, Beclin 1 and ubiquitin proteins in samples of patients with Fabry disease

The expression levels of LC3, Beclin 1 and ubiquitin proteins related to the lysosome signaling system in Fabry disease patient samples were confirmed by Western blotting.

Particularly, vascular endothelial cells were isolated from the dermal tissue obtained from the patients with Fabry disease of Example 1, to which Fabrazyme was treated. Then, the expression levels of LC3, Beclin 1 and ubiquitin proteins were confirmed by performing Western blotting in the same manner as described in Experimental Example <3-1>. At this time, anti-LC3 antibody (1:1000, Santacruz, USA), anti-Beclin 1 antibody (1:500, Santacruz, USA), and anti-ubiquitin antibody (1:1000, Santacruz, USA) were used as antibodies.

As a result, as shown in Figure 5, the expression levels of LC3, Beclin 1 and ubiquitin proteins were higher in vascular endothelial cells of patients with Fabry disease than in normal cells regardless of the Fabrazyme treatment.

From the above results, it was confirmed that the lysosomes did not function normally in patients with Fabry disease, and the characteristics of the lysosomal storage disease were not recovered even after the treatment of Fabrazyme, a therapeutic agent.

### Example 2: Preparation of vascular endothelial cells derived from patients with Fabry disease

In order to confirm the vasculopathy of patients with Fabry disease, vascular endothelial cells were differentiated from FB-iPSCs according to the following method (Figure 6).

Particularly, the FB-iPSCs prepared in Example 1 were cultured in a feeder-free state for 3 days. Thereafter, the medium was exchanged with RPMI-1640 medium containing 1% B27, to which 50 ng/ml of activin A and 20 ng/ml of BPM4 were added as growth factors, and then the cells were cultured for 2 days to differentiate into mesoderm cells. The medium was exchanged with RPMI-1640 medium containing 0.5% B27, to which 50 ng/ml of VEGFA and 50 ng/ml of bFGF were added as growth factors, and then the cells were cultured for 2 days to differentiate into vascular progenitors. CD34 positive cells capable of differentiating into vascular endothelial cells were isolated from the induced vascular progenitors by performing magnetic activated cell sorting (MACS) using CD34 magnetic beads. The isolated CD34 positive cells were further cultured for 2 days in EGM-2 medium (Lonza, USA) containing 100 ng/ml of VEGF-A and 100 ng/ml of bFGF as growth factors to differentiate into vascular endothelial cells.

As a result, as shown in Figure 7, differentiation of vascular endothelial cells was induced from FB-iPSCs, and more than 15% of cells expressing CD34 and CD31 were generated.

### Experimental Example 4: Confirmation of characteristics of vascular endothelial cells derived from patients with Fabry disease

### <4-1> Confirmation of expression of vascular endothelial cell marker protein

In order to confirm whether the vascular endothelial cells derived from Fabry disease patients were differentiated normally, the expression of the vascular endothelial cell marker proteins KDR (kinase insert domain receptor), CD31 and VE-cadherin (vascular endothelial cadherin) was confirmed using immunostaining. The experiment was carried out in the same manner as described in Experimental Example <1-1> using the vascular endothelial cells prepared in Example 2. At this time, anti-KDR antibody (1:300, Cell Signaling Technologies, USA), anti-CD31 antibody (1:300, Cell Signaling Technologies, USA), and anti-VE-cadherin antibody (1:300, Abcam, UK) were used as the primary antibodies.

Meanwhile, the distribution of cells expressing CD31 and CD105 among vascular endothelial cells was confirmed by FACS (fluorescence activated cell sorter). Particularly, the vascular endothelial cells prepared in Example 2 were treated with accutase and centrifuged to obtain a cell pellet. The cell pellet was resuspended in PBS containing 2% fetal bovine serum (FACS buffer), and the cells were filtered using a strainer having a pore size of 40 pm. The filtered cells were treated with anti-CD31 antibody (1:200, Biolegend, USA) and anti-CD105 antibody (1:200, Biolegend, USA) as FACS-specific antibodies, and reacted at 4°C for 3 minutes. Upon completion of the reaction, the cells were washed 5 times with FACS buffer, and the cell distribution was confirmed using a FACS Caliber flow cytometer (BD biosciences).

As a result, as shown in Figure 8, the vascular endothelial cell markers KDR, CD31 and VE-cadherin proteins were expressed in the vascular endothelial cells derived from patients with Fabry disease at the level in normal cells, and the expressions of CD31 and CD105 proteins were uniformly distributed. From the above results, it was confirmed that vascular endothelial cells were normally differentiated from FB-iPSCs.

### <4-2> Confirmation of morphology and vasculogenesis of vascular endothelial cells

The changes in morphology of the vascular endothelial cells derived from patients with Fabry disease and the vasculogenesis were confirmed by the following method.

First, while culturing the vascular endothelial cells prepared in Example 2 for 13 days, the morphology of cells on the 9^{th}, 11^{th}, and 13^{th} days was confirmed with a phase contrast microscope.

On the other hand, 70 µl of matrigel (corning) stock solution was put in a 96-well plate and hardened at 37°C, and then 1x10⁴ vascular endothelial cells prepared in Example 2 and EGM2 medium were added thereto to confirm the tube formation.

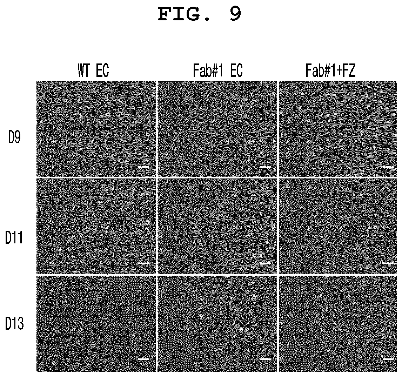

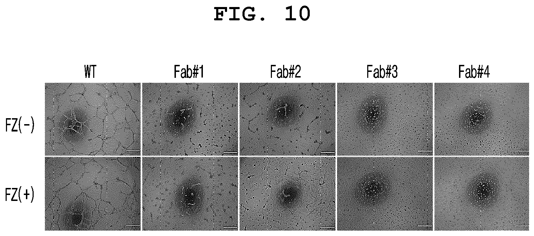

As a result, as shown in Figures 9 and 10, unlike the normal control group, the vascular endothelial cells derived from patients with Fabry disease spread wider as the culture progressed and changed into an abnormal shape, and no tube formation on the matrigel was observed. From the above results, it was confirmed that vascular endothelial cells derived from patients with Fabry disease exhibit vasculopathy.

### Experimental Example 5: Confirmation of decreased GLA activity in vascular endothelial cells derived from patients with Fabry disease

The GLA activity in the vascular endothelial cells derived from patients with Fabry disease was confirmed by the following method. The experiment was carried out in the same manner as described in Experimental Example 2 using the vascular endothelial cells prepared in Example 2.

As a result, as shown in Figure 11, unlike the normal control group, the vascular endothelial cells derived from patients with Fabry disease did not show any GLA activity.

### Experimental Example 6: Confirmation of expression of KDR protein in vascular endothelial cells derived from patients with Fabry disease

Since Fabry disease causes vascular complications, the expression of KDR protein was confirmed by the following method to confirm the vasculopathy in patients with Fabry disease.

### <6-1> Confirmation of expression site of KDR protein

The expression site of KDR protein in the vascular endothelial cells differentiated from FB-iPSCs was confirmed by immunostaining. The experiment was carried out in the same manner as described in Experimental Example <1-1> using the vascular endothelial cells prepared in Example 2. At this time, anti-KDR antibody (1:300, Cell Signaling Technologies, USA) was used as the primary antibody.

As a result, as shown in Figure 12, it was confirmed that the expression site of KDR protein in the vascular endothelial cells derived from patients with Fabry disease was different from that in the normal control group.

### <6-2> Confirmation of KDR protein signaling system

The expression levels of KDR, eNOS (endothelial Nitric oxide synthase) and TSP1 (Thrombospondin type-1) related to the KDR protein signaling system in the vascular endothelial cells differentiated from FB-iPSCs were confirmed by Western blotting.

Particularly, the vascular endothelial cells prepared in Example 2 were treated with Fabrazyme, and Western blotting was performed in the same manner as described in Experimental Example <3-1>. At this time, anti-KDR antibody (1:1000, Cell Signaling Technologies, USA), anti-eNOS antibody (1:1000, Cell Signaling Technologies, USA) and anti-TSP1 antibody (1:1000, Santacruz, USA) were used as antibodies.

As a result, as shown in Figure 13, the expression levels of KDR and eNOS proteins were decreased in the vascular endothelial cells derived from patients with Fabry disease compared to the normal control group, and the expression level of TSP1 protein was increased compared to the normal control group regardless of the Fabrazyme treatment. From the above results, it was confirmed that the expression level of TSP1 protein was increased by Fabry disease, which was not inhibited by Fabrazyme.

### Experimental Example 7: Confirmation of expression of TSP1 protein in vascular endothelial cells derived from patients with Fabry disease

To determine whether the vascular-related complications of Fabry disease are caused by overexpression of TSP1 protein, the signaling system of TSP1 protein was confirmed by the following method.

### <7-1> Confirmation of TGF-β (transforming growth factor beta) activation

In order to confirm the activation of TGF-β protein, an upper signaling system of TSP1 protein in the vascular endothelial cells differentiated from FB-iPSCs, the phosphorylation of SMAD2 protein was confirmed using Western blotting.

Particularly, the vascular endothelial cells prepared in Example 2 were treated with Fabrazyme, and Western blotting was performed in the same manner as described in Experimental Example <3-1>. At this time, anti-SMAD2 antibody (1:1000, Cell Signaling Technologies, USA) and anti-phospho-SMAD2 antibody (1:1000, Cell Signaling Technologies, USA) were used as antibodies.

As a result, as shown in Figure 14, the phosphorylation level of SMAD2 protein was increased in the vascular endothelial cells derived from patients with Fabry disease compared to the normal control group regardless of the Fabrazyme treatment. From the above results, it was confirmed that TGF-β protein was activated in the vascular endothelial cells differentiated from FB-iPSCs.

### <7-2> Confirmation of increase in expression level of TSP1 protein by activation of TGF-β

In order to confirm whether the activation of TGF-β increases the expression level of TSP1 protein, the expression levels of the related proteins including TSP-1 protein were confirmed using Western blotting.

Particularly, the vascular endothelial cells differentiated from normal iPSCs were treated with activin A to activate TGF-β, and Western blotting was performed in the same manner as described in Experimental Example <3-1>. At this time, anti-TSP1 antibody (1:1000, Santacruz, USA), anti-phospho-SMAD2 antibody (1:1000, Cell Signaling Technologies, USA), anti-SMAD2 antibody (1:1000, Cell Signaling Technologies, USA), anti-KDR antibody (1:1000, Cell Signaling Technologies, USA), anti-eNOS antibody (1:1000, Cell Signaling Technologies, USA), anti-bFGF antibody (1:1000, Bioss, USA), anti-ANG2 antibody (1:1000, Bioss, USA) and anti-vEGF antibody (1:1000, Santacruz, USA) were used as antibodies.

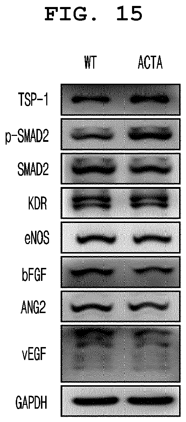

As a result, as shown in Figure 15, the expression of TSP1 protein was increased by activin A in the vascular endothelial cells differentiated from normal iPSCs. From the above results, it was confirmed that the expression level of TSP1 protein was increased by the activation of TGF-β protein.

### <7-3> Confirmation of expression location of TSP1 protein

The expression location of TSP1 protein in the vascular endothelial cells differentiated from FB-iPSCs was confirmed by immunostaining. The experiment was performed in the same manner as described in Experimental Example <1-1> using the vascular endothelial cells prepared in Example 2. Anti-TSP1 antibody (1:200, Santacruz, USA) was used as the primary antibody.

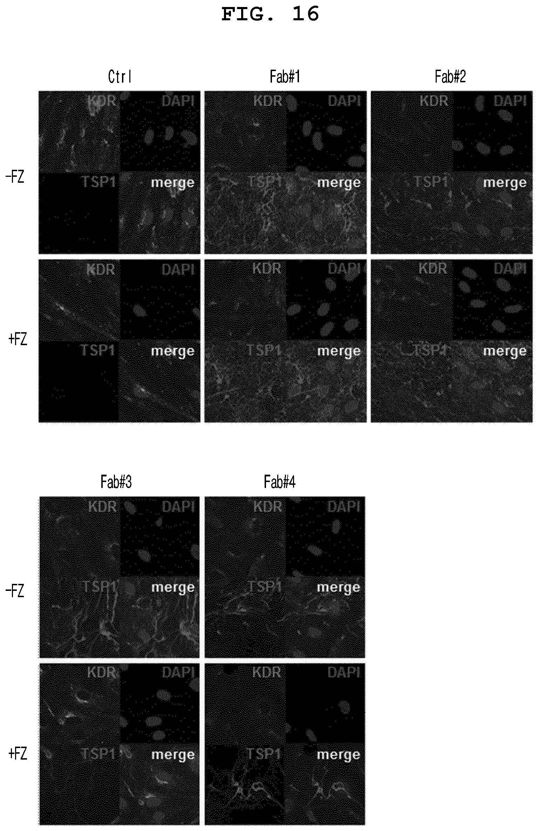

As a result, as shown in Figure 16, the increased TSP1 protein in the vascular endothelial cells derived from patients with Fabry disease was distributed throughout the cells compared to the normal control.

### Experimental Example 8: Confirmation of vasculopathy induced by Gb3 (Globotriaosylceramide 3) protein

Whether the Gb3 protein accumulated in cells and organs of patients with Fabry disease induces vasculopathy was confirmed by the following method.

### <8-1> Confirmation of changes in morphology of vascular endothelial cells

The changes in the morphology of vascular endothelial cells caused by Gb3 protein was confirmed by the following method.

Particularly, the vascular endothelial cells differentiated from normal iPSCs were treated with LysoGb3 protein (MATREYA LLC, USA) for 9 days, and then the morphology of the cells was confirmed.

As a result, as shown in Figure 17, the morphology of the normal vascular endothelial cells was changed to a widening form like the vascular endothelial cells differentiated from FB-iPSCs by Gb3 protein.

### <8-2> Confirmation of expression levels of vasculopathy-related genes

The changes in the expression levels of vasculopathy-related genes by Gb3 protein were confirmed using RT-PCR (reverse transcription polymerase chain reaction).

Particularly, the vascular endothelial cells differentiated from normal iPSCs were treated with LysoGb3 protein for 1, 3 or 5 days, and RNA was extracted from the cells using easy-BLUE (Intron). Then, RT-PCR was performed in the conventional manner to confirm the RNA expression levels of KDR, eNOS and TSP1 (Thrombospondin 1) genes related to vasculopathy.

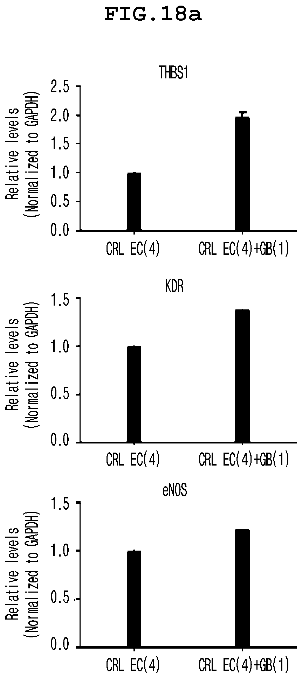

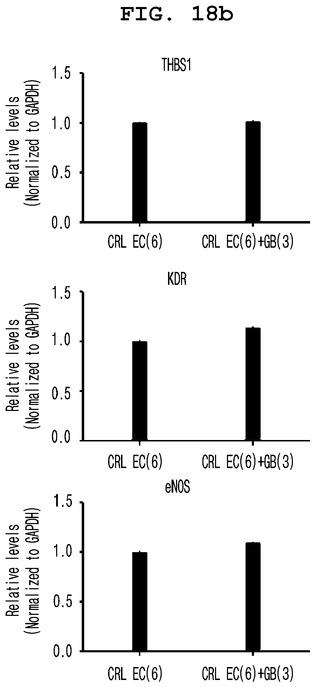

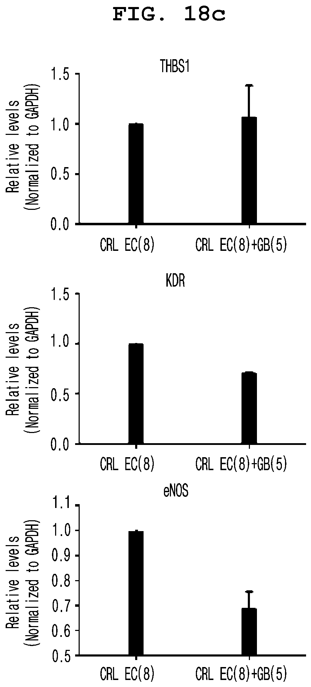

As a result, as shown in Figures 18a to 18c, the RNA expression levels of KDR, eNOS and TSP1 genes were increased 1 day after the treatment of LysoGb3 protein. The RNA expression levels were gradually decreased, and after 3 and 5 days, the RNA expression levels of KDR and eNOS genes were lower than those of the normal control cells, and the RNA expression level of TSP1 gene was similar to that of the normal control cells.

### <8-3> Confirmation of expression levels of vasculopathy-related proteins

The changes in the expression levels of vasculopathy-related proteins by Gb3 protein were confirmed using Western blotting. Particularly, the vascular endothelial cells differentiated from normal iPSCs were treated with LysoGb3 protein for 3, 6, or 9 days, and Western blotting was performed in the same manner as described in Experimental Example <3-1>. At this time, anti-TSP1 antibody (1:1000, Santacruz, USA), anti-KDR antibody (1:1000, Cell Signaling Technologies, USA), anti-eNOS antibody (1:1000, Cell Signaling Technologies, USA), anti-AKT antibody (1:1000, Cell Signaling Technologies, USA), and anti-phospho-AKT antibody (1:1000, Cell Signaling Technologies, USA) were used as antibodies.

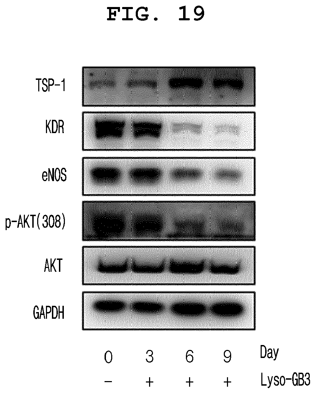

As a result, as shown in Figure 19, the expression level of TSP1 protein was increased by the treatment of LysoGb3 protein, and the expression levels of KDR and eNOS protein and the phosphorylation of AKT protein were decreased.

### <8-4> Confirmation of expression location of TSP1 protein

The expression location of TSP1 protein in the vascular endothelial cells differentiated from FB-iPSCs was confirmed by immunostaining. After treating the vascular endothelial cells differentiated from normal iPSCs with LysoGb3 protein, the experiment was performed in the same manner as described in Experimental Example <1-1>. Anti-TSP1 antibody (1:200, Santacruz, USA) was used as the primary antibody.

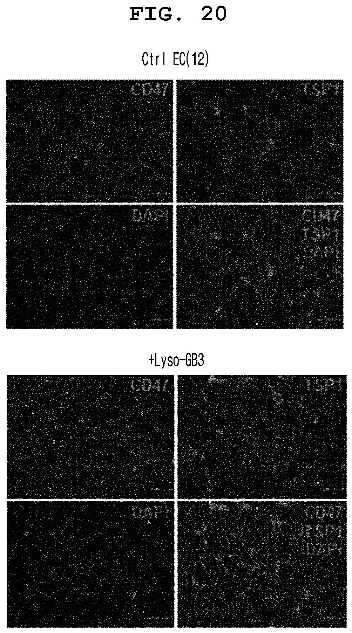

As a result, as shown in Figure 20, the TSP1 protein increased by the treatment of LysoGb3 protein was distributed throughout the cells.

From the above results, it was confirmed that the Gb3 protein is a major substance causing vasculopathy in patients with Fabry disease.

### Experimental Example 9: Confirmation of therapeutic effect of TGF-β inhibitor on vasculopathy of patients with Fabry disease

### <9-1> Confirmation of changes in morphology of vascular endothelial cells by SB431542

The changes in morphology of vascular endothelial cells by SB431542, a TGF-β inhibitor, were confirmed by the following method.

Particularly, using the control group treated with only Fabrazyme on the vascular endothelial cells prepared in Example 2, the experimental group treated with only SB431542 (Cayman Chemical, USA), and the experimental group treated with Fabrazyme and SB431542, the morphology of the cells was confirmed.

As a result, as shown in Figures 21a to 21e, the abnormal morphology of the vascular endothelial cells differentiated from FB-iPSCs was restored to normal by SB431542.

### <9-2> Confirmation of signaling system of vascular endothelial cells by SB431542

The changes in the expression levels of vasculopathy-related proteins by SB431542 were confirmed using Western blotting.

Particularly, using the control group treated with only Fabrazyme on the vascular endothelial cells prepared in Example 2 and the experimental group treated with Fabrazyme and SB431542, Western blotting was performed in the same manner as described in Experimental Example <3-1>. At this time, anti-TSP1 antibody (1:1000, Santacruz, USA), anti-KDR antibody (1:1000, Cell Signaling Technologies, USA) and anti-eNOS antibody (1:1000, Cell Signaling Technologies, USA) were used as antibodies.

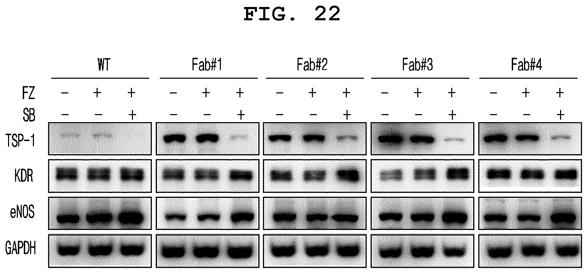

As a result, as shown in Figure 22, the expression level of TSP1 protein was decreased by SB431542, but the expression levels of KDR and eNOS proteins were increased.

### <9-3> Confirmation of vasculogenesis of vascular endothelial cells by SB431542

Vasculogenesis of vascular endothelial cells caused by SB431542 was confirmed by the following method.

Particularly, using the control group treated with only Fabrazyme on the vascular endothelial cells prepared in Example 2, the experimental group treated with only SB431542, and the experimental group treated with Fabrazyme and SB431542, The tube formation was confirmed in the same manner as described in Experimental Example <4-2>.

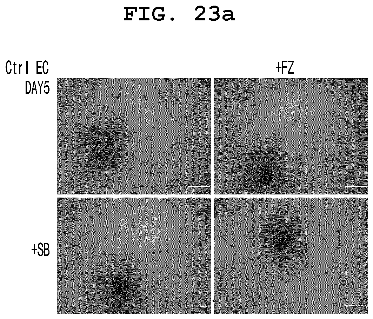

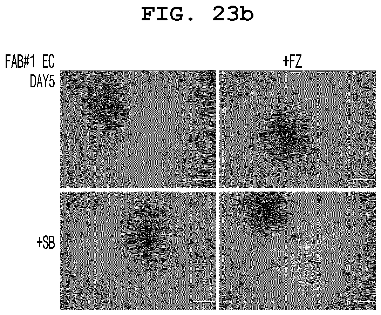

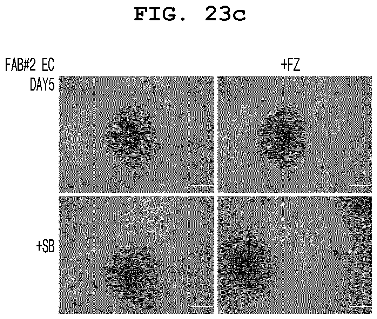

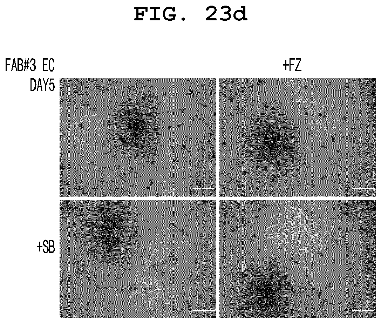

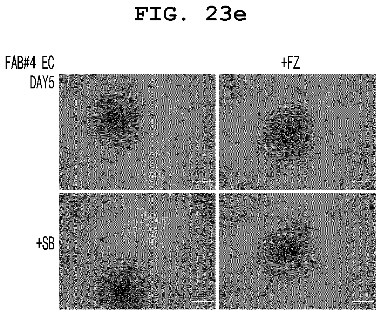

As a result, as shown in Figures 23a to 23e, the vascular endothelial cells formed a tube shape on matrigel by SB431542.

### <9-4> Confirmation of changes in morphology of vascular endothelial cells by losartan

The changes in morphology of vascular endothelial cells by losartan, a TGF-β inhibitor, were confirmed by the following method.

Particularly, using the control group treated with only Fabrazyme on the vascular endothelial cells prepared in Example 2, the experimental group treated with Fabrazyme and SB431542, and the experimental group treated with Fabrazyme and losartan (Cayman Chemical, USA), the morphology of the cells was confirmed.

As a result, as shown in Figures 24a and 24b, the abnormal morphology of the vascular endothelial cells differentiated from FB-iPSCs was restored to normal by SB431542, whereas losartan had no effect.

From the above results, SB431542 showed a therapeutic effect on vasculopathy in patients with Fabry disease, but the effect of losartan could not be confirmed.

### Example 3: Preparation of FB-iPSC isotype control cell line

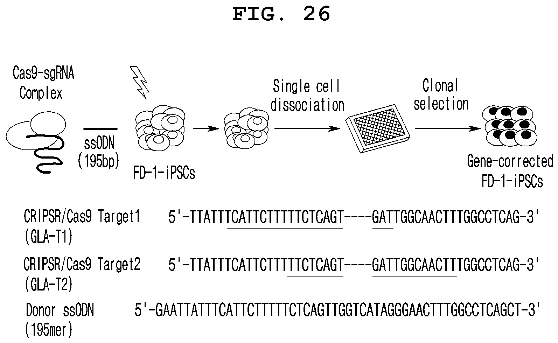

For objective experiments on vasculopathy of patients with Fabry disease, a FB-iPSC isotype control cell line was prepared using CRISPR/CAS9 system (Figures 25 and 26).

Particularly, in the FB-iPSCs prepared in Example 1, a defect in GLA gene was corrected by the conventional method using CRISPR/CAS9 system targeting the sequences shown in Table 2 below. An edited cell line was prepared using the target 2, which has a higher indel frequency for the target (Table 3), and DNA was extracted therefrom, followed by sequencing thereof.

**[Table 2]**

| CRISPR/CAS9 target gene | Nucleotide sequence (5'-3') | SEQ. ID. NO: |
|---|---|---|
| GLA (target 1) | TCATTCTTTTTCTCAGTNNNNGATTGG | 3 |
| GLA (target 2) | TTCTCAGTNNNNGATTGGCAACTTTGG | 4 |

**[Table 3]**

| | | Total reads | Insert | Deletion | Indel (%) | HDR (%) |
|---|---|---|---|---|---|---|
| Target 1 | Cas9 alone | 23233 | 0 | 3 | 3 (0.0%) | 0 (0.0%) |
| | Cas9+T1_sgRNA | 22041 | 0 | 0 | 0 (0.0%) | 0 (0.0%) |
| | Cas9+T1_sgRNA+ssODN | 24833 | 0 | 0 | 0 (0.0%) | 0 (0.0%) |
| Target 2 | Cas9 alone | 23226 | 0 | 13 | 13 (0.1%) | 0 (0.0%) |
| | Cas9+T2_sgRNA | 22636 | 339 | 2716 | 3055 (13.5%) | 0 (0.0%) |
| | Cas9+T2_sgRNA +ssODN | 21316 | 1318 | 1766 | 3084 (14.5%) | 877 (4.1%) |

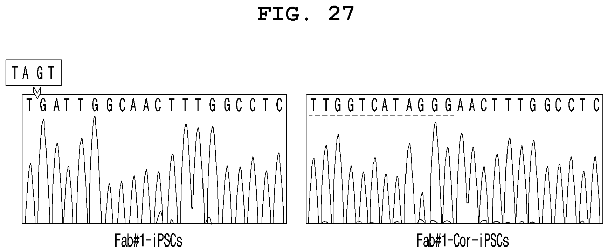

As a result, as shown in Figure 27, an isotype control cell line of FB-iPSCs with the corrected GLA gene was obtained.

### Example 4: Preparation of vascular endothelial cells differentiated from isotype control cells of FB-iPSCs

Vascular endothelial cells were prepared by differentiating the FB-iPSC isotype control cells prepared in Example 3 in the same manner as described in Example 2.

### Experimental Example 10: Confirmation of recovery of GLA activity in FB-iPSC isotype control cell line and vascular endothelial cells

Whether the GLA activity was recovered in the FB-iPSC isotype control cell line and the vascular endothelial cells differentiated therefrom was confirmed by the following method. The experiment was performed in the same manner as described in Experimental Example 2 using the FB-iPSC isotype control cell line prepared in Example 3 and the vascular endothelial cells prepared in Example 4.

As a result, as shown in Figure 28, the GLA activity was restored in the FB-iPSC isotype control cell line and the vascular endothelial cells differentiated therefrom.

### Experimental Example 11: Confirmation of recovery of vasculopathy in vascular endothelial cells differentiated from FB-iPSC isotype control cells

### <11-1> Confirmation of expression levels of vasculopathy-related proteins

The expression levels of vasculopathy-related proteins in the vascular endothelial cells differentiated from FB-iPSC isotype control cells were confirmed by Western blotting. Western blotting was performed in the same manner as described in Experimental Example <3-1> using the vascular endothelial cells prepared in Example 2 and the vascular endothelial cells prepared in Example 4 treated with GLA (Genzyme, USA). At this time, anti-TSP1 antibody (1:1000, Santacruz, USA), anti-SMAD2 antibody (1:1000, Cell Signaling Technologies, USA), anti-phospho-SMAD2 antibody (1:1000, Cell Signaling Technologies, USA), anti-KDR antibody (1:1000, Cell Signaling Technologies, USA) and anti-eNOS antibody (1:1000, Cell Signaling Technologies, USA) were used as antibodies.

As a result, as shown in Figure 29, the expression level of TSP1 protein and the phosphorylation of SMAD protein were decreased, and the expression levels of KDR and eNOS proteins were increased in the vascular endothelial cells differentiated from FB-iPSC isotype control cells, compared to the vascular endothelial cells differentiated from FB-iPSCs.

### <11-2> Confirmation of expression location of anti-vasculogenesis factor protein

The expression location of the anti-vasculogenesis factor protein in the vascular endothelial cells differentiated from FB-iPSC isotype control cells was confirmed by immunostaining.

Particularly, the experiment was performed in the same manner as described in Experimental Example <1-1> using the vascular endothelial cells prepared in Example 2 and the vascular endothelial cells prepared in Example 4 treated with GLA. Anti-VE-cadherin antibody (1:300, Abcam, UK) and anti-TSP1 antibody (1:200, Santacruz, USA) were used as the primary antibodies.

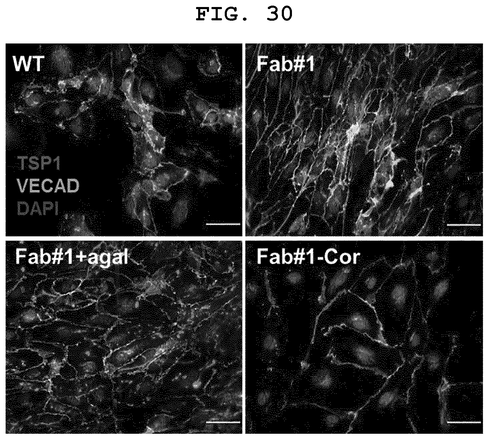

As a result, as shown in Figure 30, the distribution of TSP1 protein was decreased in the vascular endothelial cells differentiated from FB-iPSC isotype control cells compared to the vascular endothelial cells differentiated from FB-iPSCs.

### <11-3> Confirmation of vasculogenesis of vascular endothelial cells

Vasculogenesis of the vascular endothelial cells differentiated from FB-iPSC isotype control cells was confirmed by the following method. The experiment was performed in the same manner as described in Experimental Example <4-2> using the vascular endothelial cells prepared in Example 2 and the vascular endothelial cells prepared in Example 4 treated with GLA.

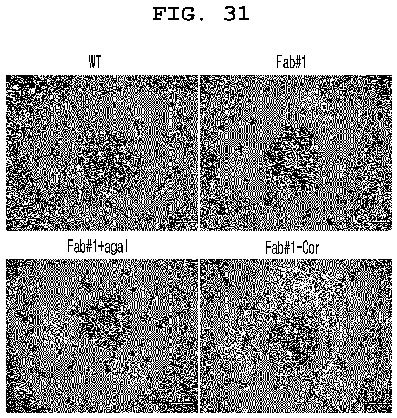

As a result, as shown in Figure 31, compared to the vascular endothelial cells differentiated from FB-iPSCs, the vascular endothelial cells differentiated from FB-iPSC isotype control cells formed a tube shape better on matrigel.

From the above results, it was confirmed that the correction of GLA gene restored vasculopathy in patients with Fabry disease.

### Example 5: Construction of TSP1 gene knocked-out FB-iPSC cell line

An FB-iPSC cell line in which TSP1 gene known as the cause of vasculopathy in Fabry disease patients was knocked out was constructed using CRISPR/CAS9 system (Figure 32).

Particularly, the TSP1 gene of the FB-iPSCs prepared in Example 1 was knocked out by the conventional method using CRISPR/CAS9 system targeting the sequences shown in Table 4 below. A knockout cell line was prepared using the target 2, which has a higher indel frequency for the target (Table 5), and DNA was extracted therefrom, followed by sequencing thereof.

**[Table 4]**

| CRISPR/CAS9 target gene | Nucleotide sequence (5'-3') | SEQ. ID. NO: |
|---|---|---|
| TSP1 (target 1) | GTCTGTGGAAGAAGCTCTCCTGG | 5 |
| TSP2 (target 2) | CTGGAGCGGAAAGACCACTCTGG | 6 |

**[Table 5]**

| | | Total reads | Insert | Deletion | Indel (%) |
|---|---|---|---|---|---|
| Target 1 | Cas9 alone | 28429 | 0 | 9 | 9 (0.0%) |
| | Cas9+T1_sgRNA | 17769 | 117 | 1154 | 1271(7.2%) |
| Target 2 | Cas9 alone | 27483 | 0 | 3 | 3 (0.0%) |
| | Cas9+T2_sgRNA | 21243 | 198 | 1880 | 2078 (9.8%) |

As a result, as shown in Figure 33, an FB-iPSC cell line in which TSP1 gene was knocked out was obtained.

### Example 6: Preparation of vascular endothelial cells differentiated from TSP1 gene knocked-out FB-iPSC cells

Vascular endothelial cells were prepared by differentiating the TSP1 gene knocked-out FB-iPSC cells prepared in Example 5 in the same manner as described in Example 2.

### Experimental Example 12: Confirmation of recovery of vasculopathy in vascular endothelial cells differentiated from TSP1 gene knocked-out FB-iPSC cells

### <12-1> Confirmation of changes in morphology of vascular endothelial cells

The changes in the morphology of the vascular endothelial cells differentiated from TSP1 gene knocked-out FB-iPSC cells were confirmed by the following method. In the experiment, the vascular endothelial cells prepared in Example 2 and the vascular endothelial cells prepared in Example 6 treated with GLA were used to confirm the cell morphology.

As a result, as shown in Figure 34, the vascular endothelial cells differentiated from FB-iPSCs showed abnormal morphology, whereas the vascular endothelial cells differentiated from TSP1 gene knocked-out FB-iPSC cells showed normal morphology.

### <12-2> Confirmation of expression level of TSP1 gene in vascular endothelial cells

The RNA expression level of TSP1 gene in the vascular endothelial cells differentiated from TSP1 gene knocked-out FB-iPSC cells was confirmed by RT-PCR. Particularly, RT-PCR was performed in the same manner as described in Experimental Example <8-2> using the vascular endothelial cells prepared in Example 2 and the vascular endothelial cells prepared in Example 6.

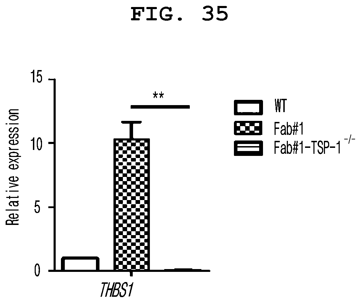

As a result, as shown in Figure 35, the RNA expression level of TSP1 gene was decreased in the vascular endothelial cells differentiated from TSP1 gene knocked-out FB-iPSC cells, compared to the normal control cells and the vascular endothelial cells differentiated from FB-iPSCs.

### <12-3> Confirmation of expression levels of vasculopathy-related proteins

The expression levels of vasculopathy-related proteins in the vascular endothelial cells differentiated from TSP1 gene knocked-out FB-iPSC cells were confirmed by Western blotting. Particularly, Western blotting was performed in the same manner as described in Experimental Example <3-1> using the vascular endothelial cells prepared in Example 2 and the vascular endothelial cells prepared in Example 6. Anti-TSP1 antibody (1:1000, Santacruz, USA), anti-KDR antibody (1:1000, Cell Signaling Technologies, USA), anti-eNOS antibody (1:1000, Cell Signaling Technologies, USA), anti-phospho-SMAD2 antibody (1:1000, Cell Signaling Technologies, USA) and anti-SMAD2 antibody (1:1000, Cell Signaling Technologies, USA) were used as antibodies.

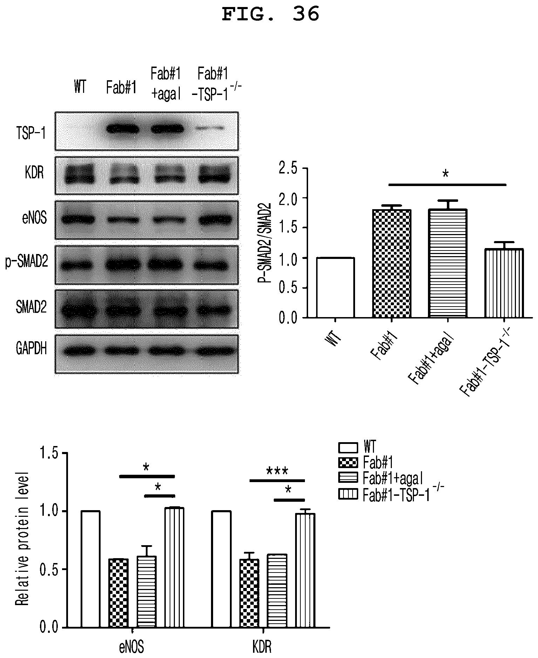

As a result, as shown in Figure 36, the expression level of TSP1 protein and the phosphorylation of SMAD protein were decreased, and the expression levels of KDR and eNOS proteins were increased in the vascular endothelial cells differentiated from TSP1 gene knocked-out FB-iPSC cells, compared to the vascular endothelial cells differentiated from FB-iPSCs.

### <12-4> Confirmation of vasculogenesis of vascular endothelial cells

Vasculogenesis of the vascular endothelial cells differentiated from TSP1 gene knocked-out FB-iPSC cells was confirmed by the following method. The experiment was performed in the same manner as described in Experimental Example <4-2> using the vascular endothelial cells prepared in Example 2 and the vascular endothelial cells prepared in Example 4 treated with GLA.

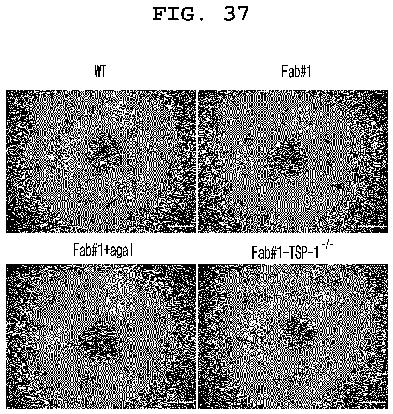

As a result, as shown in Figure 37, compared to the vascular endothelial cells differentiated from FB-iPSCs, the vascular endothelial cells differentiated from TSP1 gene knocked-out FB-iPSC cells formed a tube shape better on matrigel.

From the above results, it was confirmed that the TSP1 protein is a major protein causing vasculopathy in patients with Fabry disease, and the inhibition of its expression could cure Fabry disease.

## Claims

1. A pharmaceutical composition comprising a TSP1 (Thrombospondin1) gene expression inhibitor or a TSP1 protein activity inhibitor as an active ingredient for preventing or treating Fabry disease.

2. The pharmaceutical composition for preventing or treating Fabry disease according to claim 1, wherein the TSP1 gene is a polynucleotide composed of the nucleotide sequence represented by SEQ. ID. NO: 1.

3. The pharmaceutical composition for preventing or treating Fabry disease according to claim 1, wherein the TSP1 gene expression inhibitor includes any one or more selected from the group consisting of antisense nucleotides, siRNA (small interfering RNA), shRNA (short hairpin RNA) and ribozyme against the polynucleotide constituting TSP1 gene.

4. The pharmaceutical composition for preventing or treating Fabry disease according to claim 1, wherein the TSP1 protein is a polypeptide composed of the amino acid sequence represented by SEQ. ID. NO: 2.

5. The pharmaceutical composition for preventing or treating Fabry disease according to claim 1, wherein the TSP1 protein activity inhibitor includes any one or more selected from the group consisting of compounds, peptides, peptide mimetics, matrix analogs, aptamers, and antibodies that complementarily bind to the polypeptide constituting TSP1 protein.

6. A health functional food comprising a TSP1 gene expression inhibitor or a TSP1 protein activity inhibitor as an active ingredient for preventing or ameliorating Fabry disease.

7. A screening method of a candidate therapeutic agent for Fabry disease comprising the following steps:
1) treating a test material to a cell line expressing TSP1 protein;
2) measuring the expression level of the TSP1 protein in the cell line; and
3) selecting a test material that has reduced the expression level of the TSP1 protein compared to the control not treated with the test material.

8. The screening method of a candidate therapeutic agent for Fabry disease according to claim 7, wherein the test material of step 1) is one or more substances selected from the group consisting of peptides, proteins, non-peptidic compounds, active compounds, fermentation products, cell extracts, plant extracts, animal tissue extracts and plasma.

9. The screening method of a candidate therapeutic agent for Fabry disease according to claim 7, wherein the protein expression level of step 2) is measured by any one or more methods selected from the group consisting of enzyme-linked immunosorbent assay (ELISA), radioimmunoassay, sandwich enzyme immunoassay, western blot, immunoprecipitation, immunohistochemistry, fluoroimmunoassay and flow cytometry (FACS).

10. A screening method of a candidate therapeutic agent for Fabry disease comprising the following steps:
1) treating a test material to TSP1 protein;
2) measuring the activity level of the TSP1 protein; and
3) selecting a test material that has reduced the activity level of the TSP1 protein compared to the control not treated with the test material.

11. The screening method of a candidate therapeutic agent for Fabry disease according to claim 10, wherein the protein activity level of step 2) is measured by any one or more methods selected from the group consisting of enzyme immunoassay, fluoroimmunoassay, SDS-PAGE, mass spectrometry and protein chip.

12. A protein detection method for providing information necessary for diagnosis of Fabry disease comprising the following steps:
1) measuring the expression level of TSP1 protein in the sample derived from a subject; and
2) determining the subject with the increased expression level of the TSP1 protein compared to the control group as a subject at risk of developing Fabry disease.

13. A pharmaceutical composition comprising SB-431542 as an active ingredient for preventing or treating Fabry disease.

14. The pharmaceutical composition for preventing or treating Fabry disease according to claim 13, wherein the SB-431542 is a compound represented by formula 1 below:

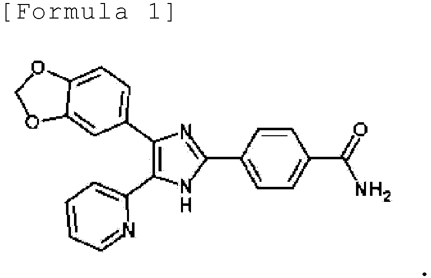

15. The pharmaceutical composition for preventing or treating Fabry disease according to claim 13, wherein the SB-431542 reduces the expression of TSP1 protein in vascular endothelial cells of patients with Fabry disease.

16. The pharmaceutical composition for preventing or treating Fabry disease according to claim 13, wherein the SB-431542 increases the expressions of KDR (kinase insert domain receptor) and eNOS (endothelial Nitric oxide synthase) proteins in vascular endothelial cells of patients with Fabry disease.

17. A health functional food comprising SB-431542 as an active ingredient for preventing or ameliorating Fabry disease.

18. The health functional food for preventing or ameliorating Fabry disease according to claim 17, wherein the SB-431542 reduces the expression of TSP1 protein and increases the expressions of KDR and eNOS proteins in vascular endothelial cells of patients with Fabry disease.
